# EUROPEAN PATENT APPLICATION

(11) **EP 2 289 515 A2**
(43) Date of publication of application: **02.03.2011**
(21) Application number: 10011899.1
(22) Date of filing: 31.10.2006
(51) Int. Cl.: A61K 31/4412, A61K 38/21, A61P 35/00

(54) **Combinations comprising a diaryl urea and an interferon**

(30) Priority: 31.10.2005 US 731277 P
(62) Divisional of application: 06827111.3
(71) Applicant: Bayer HealthCare LLC, Tarrytown, NY 10591 (US)
(72) Inventor: Wilhelm, Scott, Milford, CT 06460 (US)
(74) Representative: Riepe, Hans-Gerd

(57) **Abstract**

The present invention provides methods for treating cancer in humans and other mammals comprising administering a chemotherapeutic agent, such as an interferon, and an aryl urea compound of Formula (I) :

B-NH-C(O)-NH-L-M-L¹-(Q)₁₋₃ (I).

In Formula (I), B and L and are each, independently, optionally substituted phenyl, naphthyl, a 5 or 6 membered monocyclic heteroaryl group, or an 8 to 10 membered bicyclic heteroaryl group;
M is a bridging group.
each Q is independently C(O)R⁴, C(O)OR⁴ and C(O)NR⁴R⁵; and L' is optionally substituted phenyl, naphthyl, monocyclic heteroaryl or bicyclic heteroaryl, or a saturated or partially saturated, monocyclic or bicyclic carbocyclic moiety or heterocyclic moiety.

## Description

The present invention provides methods for treating, ameliorating, preventing, modulating, etc., cancer in humans and other mammals comprising administering effective amounts of a compound of Formula I (including pharmaceutically-acceptable salts thereof, derivatives thereof, etc.) and a chemotherapeutic agent, such as an interferon.

The aryl urea compounds employed in the methods of this invention comprise compounds of Formula I, pharmaceutically acceptable salts thereof, esters thereof, stereoisomers thereof (both isolated and in mixtures), prodrugs thereof, and any active derivatives thereof, which are collectively referred to herein as the "compounds of the invention" and the like.

Formula I is as follows:

B-NH-C(O)-NH-L-M-L¹-(Q)₁₋₃ (I)

wherein B is
(i) phenyl, optionally substituted with 1-3 substituents independently selected from the group consisting of R¹, OR¹, NR¹R², S(O)_{q}R¹, SO₂NR¹R², NR¹SO₂R², C(O)R¹, C(O)OR¹, C(O)NR¹R², NR¹C(O)R², NR¹C(O)OR², halogen, cyano, and nitro;
(ii) naphthyl, optionally substituted with 1-3 substituents independently selected from the group consisting of R¹, OR¹, NR¹R², S(O)_{q}R¹, SO₂NR¹R², NR¹SO₂R², C(O)R¹, C(O)OR¹, C(O)NR¹R², NR¹C(O)R², NR¹C(O)OR², halogen, cyano, and nitro;
(iii) a 5 or 6 membered monocyclic heteroaryl group, having 1-3 heteroatoms independently selected from the group consisting of O, N and S, optionally substituted with 1-3 substituents independently selected from the group consisting of R¹, OR¹, NR¹R², S(O)_{q}R¹, SO₂NR¹R², NR¹SO₂R², C(O)R¹, C(O)OR¹, C(O)NR¹R², NR¹C(O)R², NR¹C(O)OR², halogen, cyano, oxo, and nitro; or
(iv) an 8 to 10 membered bicyclic heteroaryl group in which the first ring is bonded to the NH of Figure I and contains 1-3 heteroatoms independently selected from the group consisting of O, N, and S, and the second ring is fused to the first ring using 3 to 4 carbon atoms. The bicyclic heteroaryl group is optionally substituted with 1-3 substituents independently selected from the group consisting of R¹, OR¹, NR¹R², S(O)_{q}R¹, SO₂NR¹R₂, NR¹SO₂R², C(O)R¹, C(O)OR¹, C(O)NR¹R², NR¹C(O)R², NR¹C(O)OR², halogen, cyano, oxo, and nitro.
L is
(i) phenyl, optionally substituted with 1-3 substituents independently selected from the group consisting of C₁-C₅ linear or branched alkyl, C₁-C₅ linear or branched haloalkyl, C₁-C₃ alkoxy, hydroxy, amino, C₁-C₃ alkylamino, C₁-C₆ dialkylamino, halogen, cyano, and nitro;
(ii) naphthyl, optionally substituted with 1-3 substituents independently selected from the group consisting of C₁-C₅ linear or branched alkyl, C₁-C₅ linear or branched haloalkyl, C₁-C₃ alkoxy, hydroxy, amino, C₁-C₃ alkylamino, C₁-C₆ dialkylamino, halogen, cyano, and nitro;
(iii) a 5 or 6 membered monocyclic heteroaryl group, having 1-3 heteroatoms independently selected from the group consisting of O, N and S, optionally substituted with 1-3 substituents independently selected from the group consisting of C₁-C₅ linear or branched alkyl, C₁-C₅ linear or branched haloalkyl, C₁-C₃ alkoxy, hydroxy, amino, C₁-C₃ alkylamino, C₁-C₆ dialkylamino, halogen, cyano, and nitro; or
(iv) an 8 to 10 membered bicyclic heteroaryl group having 1-6 heteroatoms independently selected from the group consisting of O, N and S, optionally substituted with 1-3 substituents independently selected from the group consisting of C₁-C₅ linear or branched alkyl, C₁-C₅ linear or branched haloalkyl, C₁-C₃ alkoxy, hydroxy, amino, C₁-C₃ alkylamino, C₁-C₆ dialkylamino, halogen, cyano, and nitro.
M is
(a) -(CH₂)ₘ-O-(CH₂)ₗ-,
(b) -(CH₂)ₘ₋(CH₂)ₗ-,
(c) -(CH₂)ₘ-C(O)-(CH₂)ₗ-,
(d) -(CH₂)ₘ-NR³-(CH₂)ₗ-,
(e) -(CH₂)ₘ- NR³C(O)-(CH₂)ₗ-,
(f) -(CH₂)ₘ-S-(CH₂)ₗ-,
(g) -(CH₂)m-C(O)NR³ -(CH₂)ₗ-,
(h) -(CH₂)ₘ-CF₂-(CH₂)ₗ-,
(i) -(CH₂)ₘ-CCl₂-(CH₂)ₗ-,
(j) -(CH₂)ₘ-CHF-(CH₂)ₗ-,
(k) -(CH₂)ₘ-CH(OH)-(CH₂)ₗ-;
(l) -(CH₂)ₘ-C≡C-(CH₂)ₗ-;
(m) -(CH₂)ₘ-C=C-(CH₂)ₗ-;
(n) -(CH₂)ₘ-CR⁴R⁵-(CH₂)ₗ-;
   or
(o) a single bond, where m and l are 0;
   wherein the variables m and l are integers independently selected from 0-4,
L' is
(i) phenyl, optionally substituted with 1-2 additional substituents other than Q, independently selected from the group consisting of R¹, OR¹, NR¹R², S(O)_{q}R¹, SO₂NR¹R², NR¹SO₂R², NR¹C(O)R², NR¹C(O)OR², halogen, cyano and nitro;
(ii) naphthyl, optionally substituted with 1-2 additional substituents other than Q, independently selected from the group consisting of R¹, OR¹, NR¹R², S(O)_{q}R¹, SO₂NR¹R², NR¹SO₂R², NR¹C(O)R², NR¹C(O)OR², halogen, cyano and nitro;
(iii) a 5 and 6 membered monocyclic heteroaryl group, having 1-3 heteroatoms independently selected from the group consisting of O, N and S, optionally substituted with 1-2 additional substituents other than Q, independently selected from the group consisting of R¹, OR¹, NR¹R², S(O)_{q}R¹, SO₂NR¹R², NR¹SO₂R², NR¹C(O)R², NR¹C(O)OR², halogen, cyano and nitro and also oxides (e.g. =O, -O⁻ or -OH);
(iv) an 8 to 10 membered bicyclic heteroaryl group, having 1-6 heteroatoms independently selected from the group consisting of O, N and S, optionally substituted with 1-2 additional substituents other than Q, independently selected from the group consisting of R¹, OR¹, NR¹R², S(O)_{q}R¹, SO₂NR¹R², NR¹SO₂R², NR¹C(O)R², NR¹C(O)OR², halogen, cyano and nitro and also oxides (e.g. =O, -O⁻ or-OH).
(v) a saturated and partially saturated C₃-C₆ monocyclic carbocyclic moiety optionally substituted with 1-2 additional substituents other than Q, independently selected from the group consisting of R¹, OR¹, NR¹R², S(O)_{q}R¹, SO₂NR¹R², NR'SO₂R², NR¹C(O)R², NR¹C(O)OR², halogen, cyano and, nitro;
(vi) a saturated and partially saturated C₈-C₁₀ bicyclic carbocyclic moiety, optionally substituted with 1-2 additional substituents other than Q, independently selected from the group consisting of R¹, OR¹, NR¹R², S(O)_{q}R¹, SO₂NR¹R², NR¹SO₂R², NR¹C(O)R², NR¹C(O)OR², halogen, cyano and nitro;
(vii) a saturated and partially saturated 5 and 6 membered monocyclic heterocyclic moiety, having 1-3 heteroatoms independently selected from the group consisting of O, N and S, optionally substituted with 1-2 additional substituents other than Q, independently selected from the group consisting of R¹, OR¹, NR¹R², S(O)_{q}R¹, SO₂NR¹R², NR¹SO₂R²N,R¹C(O)R², NR¹C(O)OR², halogen, cyano and nitro, and also oxides (e.g. =O, -O⁻ or -OH); or
(viii) a saturated and partially saturated 8 to 10 membered bicyclic heterocyclic moiety, having 1-6 heteroatoms independently selected from the group consisting of O, N and S, optionally substituted with 1-2 additional substituents other than Q, independently selected from the group consisting of R¹, OR¹, NR¹R², S(O)_{q}R¹, SO₂NR¹R², NR¹SO₂R², NR¹C(O)R², NR¹C(O)OR², halogen, cyano and nitro, and also oxides (e.g. =O, -O⁻ or -OH);
each Q is independently C(O)R⁴, C(O)OR⁴ and C(O)NR⁴R⁵;
wherein each R¹- R⁵ is independently selected from the group consisting of:
(a) hydrogen,
(b) C₁-C₅ linear, branched, or cyclic alkyl,
(c) phenyl,
(d) C₁-C₃) alkyl-phenyl, wherein the alkyl moiety is optionally substituted with halogen up to per-halo;
(e) up to per-halo substituted C₁-C₅ linear or branched alkyl.
(f) -(CH₂)_{q}-X, where X is a 5 or 6 membered monocyclic heterocyclic ring, containing 1-4 atoms selected from oxygen, nitrogen and sulfur, which is saturated, partially saturated, or aromatic, or a 8-10 membered bicyclic heteroaryl having 1-4 heteroatoms selected from the group consisting of O, N and S; and wherein said alkyl moiety is optionally substituted with halogen up to per-halo,
wherein each R¹ - R⁵, other than per-halo substituted C₁-C₅ linear or branched alkyl, is optionally substituted with 1-3 substituents independently selected from the group consisting of C₁-C₅ linear or branched alkyl, up to perhalo substituted C₁-C₅ linear or branched alkyl, C₁-C₃ alkoxy, hydroxy, carboxy, amino, C₁-C₃ alkylamino, C₁-C₆ dialkylamino, halogen, cyano, and nitro;
wherein the variable p is an integer selected from 0, 1, or 2 and the variable q is an integer selected from 0, 1, 2, 3, or 4.

In formula I, suitable hetaryl groups include, but are not limited to, 5-10 membered ring systems containing monocyclic and bicyclic rings, at least one of which is aromatic, in which one or more, e.g., 1-4 carbon atoms in one or more of the rings can be replaced by oxygen, nitrogen or sulfur atoms. In bicyclic ring systems, each ring can have from 3-7 atoms.

"Monocyclic heteroaryl" means an aromatic monocyclic ring having 5 to 6 ring atoms, at least one of which is a hetero atom selected from N, O and S, the remaining atoms being carbon. When more than one hetero atom is present in the moiety, they are selected independently from the other(s) so that they may be the same or different. Monocyclic heteroaryl moieties include, but are not limited to pyrrole, furan, thiophene, imidazole, pyrazole, thiazole, oxazole, isoxazole, isothiazole, triazole, tetrazole, thiadiazole, oxadiazole, pyridine, pyrimidine, pyridazine, pyrazine, and triazine.

Bicyclic heteroaryl means fused bicyclic moieties where one of the rings is chosen from the monocyclic heteroaryl rings described above and the second ring is either benzene or another monocyclic heteroaryl ring described above. When both rings in the bicyclic moiety are heteroaryl rings, they may be the same or different, as long as they are chemically accessible by means known in the art. Bicyclic heteroaryl rings include synthetically accessible 5-5, 5-6, or 6-6 fused bicyclic aromatic structures including, for example but not by way of limitation, benzoxazole (fused phenyl and oxazole), quinoline (fused phenyl and pyridine), imidazopyrimidine (fused imidazole and pyrimidine), and the like.

The phrase "5 or 6 membered heterocyclic ring, containing at least one atom selected from oxygen, nitrogen and sulfur, which is saturated, partially saturated, or aromatic" includes, by no way of limitation, tetrahydropyrane, tetrahydrofurane, 1,3-dioxolane, 1,4-dioxane, morpholine, thiomorpholine, piperazine, piperidine, piperidinone, tetrahydropyrimidone, pentamethylene sulfide, tetramethylene sulfide, dihydropyrane, dihydrofuran, dihydrothiophene, pyrrole, furan, thiophene, imidazole, pyrazole, thiazole, oxazole, isoxazole, isothiazole, triazole, pyridine, pyrimidine, pyridazine, pyrazine, triazine, and the like.

The term "C₁-C₃ alkyl-phenyl" includes, by no way of limitation, 3-phenylpropyl, 2-phenyl-1-methyl-ethyl. Substituted examples include 2-[2-chlorophenyl]ethyl, 3,4-dimethylphenyl-methyl, and the like.

Suitable substituted and unsubstituted heteroaryl groups for the compounds of this invention, such as those for B, L and L' of formula I, include, but are not limited to the following monocyclic heteroaryl groups:
2- or 3-furyl,
2- or 3-thienyl,
2- or 4-triazinyl,
1-, 2- or 3-pyrrolyl,
1-, 2-, 4- or 5-imidazolyl,
1-, 3-, 4- or 5-pyrazolyl,
2-, 4- or 5-oxazolyl,
3-, 4- or 5-isoxazolyl,
2-, 4- or 5-thiazolyl,
3-, 4- or 5-isothiazolyl,
2-, 3- or 4-pyridyl,
2-, 4-, 5- or 6-pyrimidinyl,
1,2,3-triazol-1-, -4- or-5-yl,
1,2,4-triazol-1-, -3- or-5-yl,
1- or 5-tetrazolyl,
1,2,3-oxadiazol-4- or -5-yl,
1,2,4-oxadiazol-3- or -5-yl,
1,3,4-thiadiazol-2- or -5-yl,
1,2,4-oxadiazol-3- or -5-yl,
1,3,4-thiadiazol-2- or-5-yl,
1,3,4-thiadiazol-3- or-5-yl,
1,2,3-thiadiazol-4- or -5-yl,
2-, 3-, 4-, 5- or 6-2H-thiopyranyl,
2-, 3- or 4-4H-thiopyranyl,
3- or 4-pyridazinyl, pyrazinyl, and
the following bicyclic heterocyclic groups:
   benzofuryl, benzothienyl, indolyl, benzimidazolyl, benzopyrazolyl, benzoxazolyl, benzisoxazolyl, benzothiazolyl, benzisothiazolyl, benz-1,3-oxadiazolyl, quinolinyl, isoquinolinyl, quinazolinyl, tetrahydroquinolinyl, tetrahydroisoquinolinyl, dihydrobenzofuryl, pyrazolo[3,4-b]pyrimidinyl, purinyl, benzodiazine, pterindinyl, pyrrolo[2,3-b]pyridinyl, pyrazolo[3,4-b]pyridinyl, oxazo[4,5-b]pyridinyl, imidazo[4,5-b]pyridinyl, cyclopentenopyridine, cyclohexanopyridine, cyclopentanopyrimidine, cyclohexanopyrimidine, cyclcopentanopyrazine, cyclohexanopyrazine, cyclopentanopyridiazine, cyclohexanopyridazine, cyclopentanoimidazole, cyclohexanoimidazole, cyclopentanothiophene and cyclohexanothiophene.

Suitable aryl groups which do not contain heteroatoms include, for example, phenyl and 1- and 2-naphthyl, tetrahydronaphthyl, indanyl, indenyl, benzocyclobutanyl, benzocycloheptanyl and benzocycloheptenyl.

Suitable linear alkyl groups and alkyl portions of groups, e.g., alkoxy, alkylphenyl and alkylheteroaryl etc. throughout include methyl, ethyl, propyl, butyl, pentyl, etc. Suitable branched alkyl groups include all branched isomers such as isopropyl, isobutyl, *sec*-butyl, *tert*-butyl, etc.

The term "alkoxy" means a straight or branched chain alkoxy group having saturated carbon atoms which may be linear or branched with single or multiple branching, and includes such groups as methoxy, ethoxy, n-propoxy, isopropoxy, and the like. It also includes halogenated groups such as 2,2-dichloroethoxy, trifluoromethoxy, and the like.

C₁-C₃alkylamino means methylamino, ethylamino, propylamino or isopropylamino. Examples of C₁-C₆ dialkylamino group include but are not limited to diethylamino, ethyl-isopropylamino, means methylamino, methyl-isobutylamino, dihexylamino.

Suitable halogens include F, Cl, Br, and/or I, from one to per-substitution (i.e. all H atoms on a group replaced by a halogen atom) being possible where an alkyl group is substituted by halogen, mixed substitution of halogen atom types also being possible on a given moiety. Preferred halogens are Cl, Br and F.

The term "up to perhalo substituted linear and branched alkyl," includes alkyl groups having one alkyl hydrogen replaced with halogen, alkyl groups wherein all hydrogens are replaced with halogen, alkyl groups wherein more than one but less than all hydrogens are replaced by halogen and alkyl groups having alkyl hydrogens replaced by halogen and other substituents. Examples include chloromethyl, dichloromethyl, trichloromethyl, fluoromethyl, difluoromethyl, trifluoromethyl, and the like.

The term "cycloalkyl", as used herein, refers to cyclic structures having 3-8 members in the ring such as cyclopropyl, cyclobutyl and cyclopentyl and cyclic structures having 3-8 members with alkyl substituents such that, for example, "C₃ cycloalkyl" includes methyl substituted cyclopropyl groups.

The term "saturated carbocyclic moieties" defines only the cyclic structure, i.e. cyclopentyl, cyclohexyl, etc. Any alkyl substitution on these cyclic structures is specifically identified.

Saturated monocyclic and bicyclic carbocyclic moieties include cyclopropyl, cyclobutyl, cyclopentyl, cyclohexyl, and decahydronaphthalene.

Partially saturated monocyclic and bicyclic carbocyclic moieties include cyclopentenyl, cyclohexenyl, cyclohexadienyl and tetrahydronaphthalene.

Saturated monocyclic and bicyclic heterocyclic moieties include tetrahydropyranyl, tetrahydrofuranyl, 1,3-dioxolane, 1,4-dioxanyl, morpholinyl, thiomorpholinyl, piperazinyl, piperidinyl, piperidinonyl, tetrahydropyrimidonyl, pentamethylene sulfide and tetramethylene sulfide.

Partially saturated monocyclic and bicyclic heterocyclic moieties include dihydropyranyl, dihydrofuranyl, dihydrothienyl, dihydropiperidinyl, and dihydropyrimidonyl.

When any moiety is "substituted", it can have up to the highest number of indicated substituents, and each substituent can be located at any available position on the moiety and can be attached through any available atom on the substituent. "Any available position" means any position on the moiety that is chemically accessible through means known in the art or taught herein and that does not create an unduly unstable molecule. When there are two or more substituents on any moiety, each substituent is defined independently of any other substituent and can, accordingly, be the same or different.

The term "optionally substituted" means that the moiety so modified may be either unsubstituted, or substituted with the identified substituent(s).

It is understood that where L' is pyridine, the term "hydroxy" as a pyridine substituent includes 2-, 3-, and 4-hydroxypyridine, but also includes those structures referred to in the art as 1-oxo-pyridine and 1-hydroxy-pyridine.

Where the plural form of the word compounds, salts, and the like, is used herein, this is taken to mean also a single compound, salt, or the like.

The substituted structures of B and L' are preferably each, independently, selected from the group consisting of
methyl, trifluoromethyl, ethyl, n-propyl, n-butyl, n-pentyl, isopropyl, *tert-*butyl, sec-butyl, isobutyl, cyclopropyl, cyclobutyl, cyclopentyl, methoxy, ethoxy, propoxy, Cl, Br and F, cyano, nitro, hydroxy, amino, methylamino, dimethylamino, ethylamino and diethylamino.

Other substituents for B and L' particularly include:
phenyl, pyridinyl, pyrimidinyl, chlorophenyl, dichlorophenyl, bromophenyl, dibromophenyl, chloropyridinyl, bromopyridinyl, dichloropyridinyl, dibromopyridinyl methylphenyl, methylpyridinyl quinolinyl, isoquinolinyl, isoindolinyl, pyrazinyl, pyridazinyl, pyrrolinyl, imidazolinyl, thienyl, furyl, isoxazolinyl, isothiazolinyl, benzopyridinyl, benzothiazolyl,
C₁-C₅ acyl;
NH(C₁-C₅ alkyl, phenyl or pyridinyl), such as aminophenyl;
   N(C₁-C₅ alkyl)(C₁-C₅ alkyl, phenyl or pyridinyl), such as diethylamino and dimethyl amino;
S(O)q (C₁-C₅ alkyl); such as methanesulfonyl;
S(O)q H;
SO₂NH₂;
SO₂NH(C₁-C₅ alkyl);
SO₂N(C₁-C₅ alkyl)(C₁-C₅ alkyl);
NHSO₂(C₁-C₅ alkyl); N(C₁-C₃ alkyl) SO₂(C₁-C₅ alkyl);
CO(C₁-C₆ alkyl or phenyl);
C(O)H;
C(O)O(C₁-C₆ alkyl or phenyl), such as C(O)OCH₃, -C(O)OCH₂CH₃, - C(O)OCH₂CH₂CH₃;
C(O)OH;
C(O)NH₂ (carbamoyl);
   C(O)NH(C₁-C₆ alkyl or phenyl), such as N-methylethyl carbamoyl, N-methyl carbamoyl, N-ethylcarbamoyl, or N-dimethylamino ethyl carbamoyl;
C(O)N(C₁-C₆ alkyl or phenyl)(C₁-C₆ alkyl, phenyl or pyridinyl), such as N-dimethylcarbamoyl;
C(N(C₁-C₆ alkyl)) (C₁-C₅ alkyl);
NHC(O)(C₁-C₆ alkyl or phenyl) and
N(C₁-C₅ alkyl,)C(O)(C₁-C₅ alkyl).

Each of the above substituents is optionally partially or fully halogenated, such as difluoromethyl sulfonyl.

An embodiment of this invention includes the administration of compounds of this invention wherein in formula I, L, B and L' follow one of the following of combinations:
B= phenyl, L=phenyl and L' is phenyl, pyridinyl, quinolinyl, isoquinolinyl or not present,
B= phenyl, L=pyridinyl and L' is phenyl, pyridinyl, quinolinyl, isoquinolinyl or not present,
B=phenyl, L = naphthyl and L' is phenyl, pyridinyl, quinolinyl, isoquinolinyl or not present,
B=pyridinyl, L= phenyl and L' is phenyl, pyridinyl, quinolinyl, isoquinolinyl or not present,
B=pyridinyl, L= pyridinyl and L' is phenyl, pyridinyl, quinolinyl, isoquinolinyl or not present,
B=pyridinyl, L= naphthyl and L' is phenyl, pyridinyl, quinolinyl, isoquinolinyl or not present,
B=isoquinolinyl, L= phenyl and L' is phenyl, pyridinyl, quinolinyl, isoquinolinyl or not present,
B= isoquinolinyl, L= pyridinyl and L' is phenyl, pyridinyl, quinolinyl, isoquinolinyl or not present,
B= isoquinolinyl, L= naphthyl and L' is phenyl, pyridinyl, quinolinyl, isoquinolinyl or not present,
B= quinolinyl, L= phenyl and L' is phenyl, pyridinyl, quinolinyl, isoquinolinyl or not present,
B= quinolinyl, L= pyridinyl and L' is phenyl, pyridinyl, quinolinyl, isoquinolinyl or not present,
B= quinolinyl, L= naphthyl and L' is phenyl, pyridinyl, quinolinyl, isoquinolinyl or not present.

The structure M of formula I is preferably -O-, a single bond, -S-, -NH-, - N(CH₃)-, -NHCH₂-, - NC₂H₄-, -CH₂-, -C(O)-, -CH(OH)-, -NHC(O)N(CH₃)CH₂-,-N(CH₃)C(O)N(CH₃)CH₂-, -CH₂C(O)N(CH₃)-, -C(O)N(CH₃)CH₂-, -NHC(O)-, - N(CH₃)C(O)-, -C(O)N(CH₃)-, -C(O)NH-, -CH₂O-, -CH₂S-, -CH₂N(CH₃)-, -OCH₂-, - CHF-, -CF₂-,-CCl₂-, -S-CH₂- , and -N(CH₃)CH₂-.

Compounds of the invention of particular interest include those of formula I wherein L', L, M and Q are as defined above and B is phenyl, optionally substituted with 1-4 halogen.

Compounds of the invention of particular interest also include those of formula I wherein L, L' and Q are as defined above, M is -O- and B is phenyl, optionally substituted with 1-4 halogen.

Compounds of the invention of particular interest also include those of formula I wherein B is phenyl or pyridyl, optionally substituted with 1-6 substituents independently selected from the group consisting of R¹ and halogen, L' and Q are as defined above, M is -O- and L is phenyl, optionally substituted with 1-4 halogen.

Compounds of the invention of particular interest also include those of formula I wherein B is phenyl, optionally substituted with 1-6 substituents independently selected from the group consisting of R¹ and halogen, L' and Q are as defined above, M is -O- and L is phenyl, optionally substituted with 1-4 halogen.

Compounds of the invention of particular interest also include those of formula I wherein B is 4-chloro(2-trifluoromethyl)phenyl, optionally substituted by the group consisting of R¹ and halogen, L' and Q are as defined above, M is -O- and L is phenyl, optionally substituted with 1-4 halogen.

One of ordinary skill in the art will recognize that some of the compounds of Formula (I) can exist in different geometrical isomeric forms. It is intended that all such configurations (including enantiomers and diastereomers), are included within the scope of the present invention. A number of the compounds of Formula I possess asymmetric centers, depending on the location a nature of various substituents and can therefore exist in racemic and optically active forms as well as in the form of racemic or non-racemic mixtures thereof, and in the form of diastereomers and diastereomeric mixtures. Asymmetric carbon atoms may be present in the (*R*) or (*S*) configuration or (*R,S*) configuration. In certain instances, asymmetry may also be present due to restricted rotation about a given bond, for example, the central bond adjoining two substituted aromatic rings of the specified compounds. All of these compounds, including *cis* isomers, *trans* isomers, diastereomic mixtures, racemates, non-racemic mixtures of enantiomers, substantially pure, and pure enantiomers, are considered to be within the scope of the compounds of this invention and are collectively referred to when reference is made to compounds of this invention. Therefore, the methods of the present invention encompass the use of any isolated racemic or optically active form of compounds described in Formula I which possess anticancer or anti hyperproliferative activity.

Methods of separation of enantiomeric and diastereomeric mixtures are well known to one skilled in the art. The optical isomers can be obtained by resolution of the racemic mixtures according to conventional processes, for example, by the formation of diastereoisomeric salts using an optically active acid or base. Examples of appropriate acids are tartaric, diacetyltartaric, dibenzoyltartaric, ditoluoyltartaric and camphorsulfonic acid. Mixtures of diastereoisomers can be separated into their individual diastereomers on the basis of their physical chemical differences by methods known to those skilled in the art, for example, by chromatography or fractional crystallization. The optically active bases or acids are liberated from the separated diastereomeric salts.

Another process for separation of optical isomers involves the use of a chiral chromatography column (e.g., chiral HPLC columns) optimally chosen to maximize the separation of the enantiomers. Suitable chiral HPLC columns are manufactured by Diacel, e.g., Chiracel OD and Chiracel OJ. The optically active compounds of Formula (I) can likewise be obtained by utilizing optically active starting materials.

The present invention encompasses any separated, isolated, pure or partially purified isomers or racemic mixtures of the compounds of formula I which possess activity in treating cancers. The term stereoisomer is understood to encompass diastereoisomers, enantiomers, geometric isomers, etc.

Preferred compounds are those with the absolute configuration of the compound of Formula I which produce the more desirable biological activity are also included within the scope of the present invention. The purification of said isomers and the separation of said isomeric mixtures can be accomplished by standard techniques known in the art. Herein, substantially pure enantiomers is intended to mean that no more than 5% w/w of the corresponding opposite enantiomer is present.

Pharmaceutically-acceptable salts of these compounds, as well as commonly used prodrugs of these compounds, are also within the scope of the invention. The term "pharmaceutically acceptable salt" refers to a relatively non-toxic, inorganic, or organic acid addition salt of a compound of the present invention. For example, see S. M. Berge, et al. "Pharmaceutical Salts," J. Pharm. Sci. 1977, 66, 1-19.

Suitable salts are especially the pharmaceutically acceptable salts of compounds of formula (I) or such as, for example, organic or inorganic acid addition salts of compounds of formula (I). Suitable acid addition salts include acetate, adipate, alginate, ascorbate, aspartate, benzoate, benzenesulfonate, bisulfate, butyrate, citrate, camphorate, camphorsulfonate, cinnamate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, itaconate, lactate, maleate, mandelate, methanesulfonate, 2-naphthalenesulfonate, nicotinate, nitrate, oxalate, pamoate, pectinate, persulfate, 3-phenylpropionate, picrate, pivalate, propionate, succinate, sulfonate, tartrate, thiocyanate, tosylate, and undecanoate. Suitable inorganic acids include but are not limited to halogen acids (such as hydrochloric acid and hydrobromic acid), sulfuric acid, or phosphoric acid. Suitable organic acids include but are not limited to carboxylic, phosphonic, sulfonic, or sulfamic acids, with examples including acetic acid, propionic acid, octanoic acid, decanoic acid, trifluoroacetic acid, dodecanoic acid, glycolic acid, lactic acid, 2- or 3-hydroxybutyric acid, γ-aminobutyric acid (GABA), gluconic acid, glucosemonocarboxylic acid, benzoic acid, salicylic acid, phenylacetic acid and mandelic acid, fumaric acid, succinic acid, adipic acid, pimelic acid, suberic acid, azeiaic acid, maleic acid, tartaric acid, citric acid, glucaric acid, galactaric acid, amino acids (such as glutamic acid, aspartic acid, N-methylglycine, acetytaminoacetic acid, N-acetylasparagine or N-acetylcysteine), pyruvic acid, acetoacetic acid, methanesulfonic acid, tri-fluoromethane sulfonic acid, 4-toluene sulfonic acid, benzenesulfonic acid, 1-naphthalenesulfonic acid, 2-naphthalenesulfonic acid, phosphoserine, and 2- or 3-glycerophosphoric acid.

In addition, pharmaceutically acceptable salts include acid salts of inorganic bases, such as salts containing alkaline cations (e.g., Li⁺ Na⁺ or K⁺), alkaline earth cations (e.g., Mg⁺², Ca⁺² or Ba⁺²), the ammonium cation, as well as acid salts of organic bases, including aliphatic and aromatic substituted ammonium, and quaternary ammonium cations, such as those arising from protonation or peralkylation of triethylamine, *N,N*-diethylamine, *N,N*-dicyclohexylamine, lysine, pyridine, *N,N* dimethylaminopyridine (DMAP), 1,4-diazabiclo[2.2.2]octane (DABCO), 1,5-diazabicyclo[4.3.0]non-5-ene (DBN) and 1,8-diazabicyclo[5.4.0]undec-7-ene (DBU).

Base salts include alkali metal salts such as potassium and sodium salts, alkaline earth metal salts such as calcium and magnesium salts, and ammonium salts with organic bases such as dicyclohexylamine and N-methyl-D-glucamine. Additionally, basic nitrogen containing groups may be quaternized with such agents as lower alkyl halides such as methyl, ethyl, propyl, and butyl chlorides, bromides and iodides; dialkyl sulfates like dimethyl, diethyl, and dibutyl sulfate; and diamyl sulfates, long chain halides such as decyl, lauryl, myristyl and strearyl chlorides, bromides and iodides, aralkyl halides like benzyl and phenethyl bromides and others.

The esters of appropriate compounds of this invention are well-tolerated, pharmaceutically acceptable esters such as alkyl esters including methyl, ethyl, propyl, isopropyl, butyl, isobutyl or pentyl esters. Additional esters such as phenyl-C₁-C₅ alkyl may be used, although methyl ester is preferred.

The formation of prodrugs is well known in the art in order to enhance the properties of the parent compound; such properties include solubility, absorption, biostability and release time (see *"*Pharmaceutical Dosage Form and Drug Delivery Systems" (Sixth Edition), edited by Ansel et al., published by Williams & Wilkins, pages 27-29, (1995) which is hereby incorporated by reference). Commonly used prodrugs of the disclosed oxazolyl-phenyl-2,4-diamino-pyrimidine compounds are designed to take advantage of the major drug biotransformation reactions and are also to be considered within the scope of the invention. Major drug biotransformation reactions include N-dealkylation, O-dealkylation, aliphatic hydroxylation, aromatic hydroxylation, N-oxidation, S-oxidation, deamination, hydrolysis reactions, glucuronidation, sulfation and acetylation (see Goodman and Gilman's The Pharmacological Basis of Therapeutics (Ninth Edition), editor Molinoff et al., pub. by McGraw-Hill, pages 11-13, (1996), which is hereby incorporated by reference).

The compounds of the present invention can also modulate one or more of the following processes, including, but not limited to, e.g., cell growth (including, e.g., differentiation, cell survival, and/or proliferation), tumor cell growth (including, e.g., differentiation, cell survival, and/or proliferation), tumor regression, endothelial cell growth (including, e.g., differentiation, cell survival, and/or proliferation), angiogenesis (blood vessel growth), lymphangiogenesis (lymphatic vessel growth), and/or hematopoiesis (e.g., T- and B-cell development, dendritic cell development, etc.).

Methods include modulating tumor cell proliferation, including inhibiting cell proliferation. The latter indicates that the growth and/or differentiation of tumor cells is reduced, decreased, diminished, slowed, etc. The term "proliferation" includes any process which relates to cell growth and division, and includes differentiation and apoptosis.

Any tumor or cancer can be treated, including, but not limited to, cancers having one or more mutations in raf, VEGFR-2, VEGFR-3, PDGFR-beta, Flt-3, and/or ras, as well as any upstream or downstream member of the signaling pathways of which they are a part. A cancer can be treated with a compound of the present invention irrespective of the mechanism which is responsible for it. Cancers of any organ can be treated, including cancers of, but are not limited to, e.g., colon, pancreas, breast, prostate, bone, liver, kidney, lung, testes, skin, pancreas, stomach, colorectal cancer, renal cell carcinoma, hepatocellular carcinoma, melanoma, etc.

Examples of breast cancer include, but are not limited to, invasive ductal carcinoma, invasive lobular carcinoma, ductal carcinoma in situ, and lobular carcinoma in situ.

Examples of cancers of the respiratory tract include, but are not limited to, small-cell and non-small-cell lung carcinoma, as well as bronchial adenoma and pleuropulmonary blastoma.

Examples of brain cancers include, but are not limited to, brain stem and hypophtalmic glioma, cerebellar and cerebral astrocytoma, medulloblastoma, ependymoma, as well as neuroectodermal and pineal tumor.

Tumors of the male reproductive organs include, but are not limited to, prostate and testicular cancer. Tumors of the female reproductive organs include, but are not limited to, endometrial, cervical, ovarian, vaginal, and vulvar cancer, as well as sarcoma of the uterus.

Tumors of the digestive tract include, but are not limited to, anal, colon, colorectal, esophageal, gallbladder, gastric, pancreatic, rectal, small-intestine, and salivary gland cancers.

Tumors of the urinary tract include, but are not limited to, bladder, penile, kidney, renal pelvis, ureter, and urethral cancers.

Eye cancers include, but are not limited to, intraocular melanoma and retinoblastoma.

Examples of liver cancers include, but are not limited to, hepatocellular carcinoma (liver cell carcinomas with or without fibrolamellar variant), cholangiocarcinoma (intrahepatic bile duct carcinoma), and mixed hepatocellular cholangiocarcinoma.

Skin cancers include, but are not limited to, squamous cell carcinoma, Kaposi's sarcoma, malignant melanoma, Merkel cell skin cancer, and non-melanoma skin cancer.

Head-and-neck cancers include, but are not limited to, laryngeal, hypopharyngeal, nasopharyngeal, and/or oropharyngeal cancers, and lip and oral cavity cancer.

Lymphomas include, but are not limited to, AIDS-related lymphoma, non-Hodgkin's lymphoma, cutaneous T-cell lymphoma, Hodgkin's disease, and lymphoma of the central nervous system.

Sarcomas include, but are not limited to, sarcoma of the soft tissue, osteosarcoma, malignant fibrous histiocytoma, lymphosarcoma, and rhabdomyosarcoma.

Leukemias include, but are not limited to, acute myeloid leukemia, acute lymphoblastic leukemia, chronic lymphocytic leukemia, chronic myelogenous leukemia, and hairy cell leukemia.

In addition to inhibiting the proliferation of tumor cells, compounds of the present invention can also cause tumor regression, e.g., a decrease in the size of a tumor, or in the extent of cancer in the body.

The present invention also relates to methods of modulating angiogenesis and/or lymphangiogenesis in a system comprising cells, comprising administering to the system an effective amount of a compound described herein. A system comprising cells can be an in vivo system, such as a tumor in a patient, isolated organs, tissues, or cells, in vitro assays systems (CAM, BCE, etc), animal models (e.g., in vivo, subcutaneous, cancer models), hosts in need of treatment (e.g., hosts suffering from diseases having angiogenic and/or lymphangiogenic component, such as cancer), etc. Preferred compounds of the present invention inhibit angiogenesis and/or lymphangiogenesis, e.g., the formation of new blood vessels.

The invention also relates to methods for treating, preventing, modulating, etc., diseases and conditions, comprising administering a compound of this invention with another active agent with the concurrent or intermittent administration of another active agent over the same total time period.

Optional anti-hyper-proliferative agents which can be added to the composition include but are not limited to compounds listed on the cancer chemotherapy drug regimens in the 11th Edition of the Merck Index, (1996), which is hereby incorporated by reference, such as asparaginase, bleomycin, carboplatin, carmustine, chlorambucil, cisplatin, colaspase, cyclophosphamide, cytarabine, dacarbazine, dactinomycin, daunorubicin, doxorubicin (adriamycine), epirubicin, etoposide, 5-fluorouracil, hexamethylmelamine, hydroxyurea, ifosfamide, irinotecan, leucovorin, lomustine, mechlorethamine, 6-mercaptopurine, mesna, methotrexate, mitomycin C, mitoxantrone, prednisolone, prednisone, procarbazine, raloxifen, streptozocin, tamoxifen, thioguanine, topotecan, vinblastine, vincristine, and vindesine.

Other anti-hyper-proliferative agents suitable for use with the composition of the invention include, but are not limited to, those compounds acknowledged to be used in the treatment of neoplastic diseases in Goodman and Gilman's The Pharmacological Basis of Therapeutics (Ninth Edition), editor Molinoff et al publ. by McGraw-Hill, pages 1225-1287, (1996), which is hereby incorporated by reference, such as aminoglutethimide, L-asparaginase, azathioprine, 5-azacytidine cladribine, busulfan, diethylstilbestrol, 2', 2'-difluorodeoxycytidine, docetaxel, erythrohydroxynonyladenine, ethinyl estradiol, 5-fluorodeoxyuridine, 5-fluorodeoxyuridine monophosphate, fludarabine phosphate, fluoxymesterone, flutamide, hydroxyprogesterone caproate, idarubicin, interferon (e.g., interferon-alpha, interferon-beta, interferon gamma, and subtypes thereof, including interferon-alpha-2a, interferon-2b, and -2c), medroxyprogesterone acetate, megestrol acetate, melphalan, mitotane, paclitaxel, pentostatin, N-phosphonoacetyl-L-aspartate (PALA), plicamycin, semustine, teniposide, testosterone propionate, thiotepa, trimethylmelamine, uridine, and vinorelbine.

Interferons are preferably administered with a Formula I compound of the present invention, prefereably an interferon-alpha. There are over 20 different variants of interferon-alpha which differ in molecular weight, sequence, and degree of glycosylation. Itnerferon-alpha-2a and -2b differ by about one amino acid. Interferon-alpha-2a is available commercially as a recombinant polypeptide.

For purposes of administration, any interferon can be administered, including interferon-alpha (and subtypes thereof), interferon-beta, interferon-gamma, and interferon-tau. Interferons can be administered routinely, e.g., concomitantly, sequentially, or according to its conventional dosing regime, in combination with a compound of the present invention. For example, interferon-alpha-2a can be administered in about 1, 3, 6, 9, or more MIU (million international units) per dose, about three times per week or more, two times per week or more, one a week or more, etc. A compound of the present invention, such as sorafinib can be administered along with it, e.g., in about 200, 300, 400, 500, or 600 mg bid (twice a day), These amounts can be adjusted routinely depending on the subject's response to the combination therapy.

Combination therapy as described above can be administered to subjects having a cancer (e.g., renal cell carcinoma, melanoma, hepatocellular cancer, breast cancer, CML, AML, etc) who have become refractory to a standard therapy. In some cases, the agent to which the patient is no longer responsive to can be continued, where the Formula I compound restores sensitivity to the compound.

The diarylureas of the present invention can be combined with other chemotherapeutic agents as mentioned above to provide (1) better efficacy in reducing the growth of a tumor or even eliminate the tumor as compared to administration of either agent alone, (2) provide for the administration of lesser amounts of the administered chemotherapeutic agents, (3) provide for a chemotherapeutic treatment that is well tolerated in the patient with fewer deleterious pharmacological complications than observed with single agent chemotherapies and certain other combined therapies, (4) provide for treating a broader spectrum of different cancer types in mammals, especially humans, (5) provide for a higher response rate among treated patients, (6) provide for a longer survival time among treated patients compared to standard chemotherapy treatments, (7) provide a longer time for tumor progression, and/or (8) yield efficacy and tolerability results at least as good as those of the agents used alone, compared to known instances where other cancer agent combinations produce antagonistic effects. The amounts administered can be synergistic, e.g., where the joint action of the agents is such that the combined effect is greater than the algebraic sum of their individual effects.

Compounds of the present invention can be administered in any form by any effective route, including, e.g., oral, parenteral, enteral, intravenous, intraperitoneal, topical, transdermal (e.g., using any standard patch), ophthalmic, nasally, local, non-oral, such as aerosal, inhalation, subcutaneous, intramuscular, buccal, sublingual, rectal, vaginal, intra-arterial, and intrathecal, etc. They can be administered alone, or in combination with any ingredient(s), active or inactive. They can be administered in any effective dosage, e.g., from about 0.1 to about 200 mg/kg of total body weight.

Compounds can be administered by the oral route using the pharmaceutical composition of the present invention will generally range, based on body weight, from about 0.01 mg/kg to about 50 mg/kg; from about 1 mg/kg to about 40 mg/kg; from about 5 mg/kg to about 30 mg/kg; from about 10 to about 25 mg/kg; about 10 mg/kg; about 20 mg/kg; about 25 mg/kg; about 30 mg/kg; etc.

Any suitable dosing interval can be used in accordance with the present invention. For example, the compound can be administered once, twice (BID), three, four, etc., times a day. For example, about 100, about 200, about 400 mg, about 500 mg, about 600 mg, or about 800 mg can be administered one, twice, or three times daily.

Compounds can be administered at any suitable time. For example, it can be administered routinely as other chemotherapeutic agents; it can be administered as a bolus prior to a surgical intervention; prior to or after radiation, radiofrequency ablation and other energy treatments; post-operatively; pre-operatively; etc.

The present invention relates to a method for using the compounds described above (Compounds of Formula I), including salts and esters thereof and compositions thereof, to treat mammalian hyper-proliferative disorders. This method comprises administering to a mammal in need thereof, including a human, an amount of a compound of this invention, or a pharmaceutically acceptable salt or ester thereof, which is effective to treat the disorder. Hyper-proliferative disorders include but are not limited to solid tumors, such as cancers of the breast, respiratory tract, brain, reproductive organs, digestive tract, urinary tract, eye, liver, skin, head and neck, thyroid, parathyroid and their distant metastases. Those disorders also include lymphomas, sarcomas, and leukemias.

Synthetic transformations that may be employed in the synthesis of compounds of Formula I and in the synthesis of intermediates involved in the synthesis of compounds of Formula I are known by or accessible to one skilled in the art. Collections of synthetic transformations may be found in compilations, such as:
- J. March. Advanced Organic Chemistry, 4th ed.; John Wiley: New York (1992)
- R.C. Larock. Comprehensive Organic Transformations, 2nd ed.; Wiley-VCH: New York (1999)
- F.A. Carey; R.J. Sundberg. Advanced Organic Chemistry, 2nd ed.; Plenum Press: New York (1984)
- T.W. Greene; P.G.M. Wuts. Protective Groups in Organic Synthesis, 3rd ed.; John Wiley: New York (1999)
- L.S. Hegedus. Transition Metals in the Synthesis of Complex Organic Molecules, 2nd ed.; University Science Books: Mill Valley, CA (1994)
- L.A. Paquette, Ed. The Encyclopedia of Reagents for Organic Synthesis; John Wiley: New York (1994)
- A.R. Katritzky; O. Meth-Cohn; C.W. Rees, Eds. Comprehensive Organic Functional Group Transformations; Pergamon Press: Oxford, UK (1995)
- G. Wilkinson; F.G A. Stone; E.W. Abel, Eds. Comprehensive Organometallic Chemistry; Pergamon Press: Oxford, UK (1982)
- B.M. Trost; I. Fleming. Comprehensive Organic Synthesis; Pergamon Press: Oxford, UK (1991)
- A.R. Katritzky; C.W. Rees Eds. Comprehensive Heterocylic Chemistry; Pergamon Press: Oxford, UK (1984)
- A.R. Katritzky; C.W. Rees; E.F.V. Scriven, Eds. Comprehensive Heterocylic Chemistry II; Pergamon Press: Oxford, UK (1996)
- C. Hansch; P.G. Sammes; J.B. Taylor, Eds. Comprehensive Medicinal Chemistry: Pergamon Press: Oxford, UK (1990).

In addition, recurring reviews of synthetic methodology and related topics include Organic Reactions; John Wiley: New York; Organic Syntheses; John Wiley: New York; Reagents for Organic Synthesis: John Wiley: New York; The Total Synthesis of Natural Products; John Wiley: New York; The Organic Chemistry of Drug Synthesis; John Wiley: New York; Annual Reports in Organic Synthesis; Academic Press: San Diego CA; and Methoden der Organischen Chemie (Houben-Weyl); Thieme: Stuttgart, Germany. Furthermore, databases of synthetic transformations include *Chemical Abstracts*, which may be searched using either CAS OnLine or SciFinder, *Handbuch der Organischen* Chemie(Beilstein), which may be searched using SpotFire, and REACCS.

### General Preparative Methods

The diaryl ureas of Formula I may be prepared by the use of known chemical reactions and procedures, some from starting materials which are commercially available. Nevertheless, general preparative methods are provided below to aid one skilled in the art in synthesizing these compounds, with more detailed examples being provided in the Experimental section which follows.

Substituted anilines may be generated using standard methods (March. Advanced Organic Chemistry, 3rd Ed.; John Wiley: New York (1985). Larock. Comprehensive Organic Transformations; VCH Publishers: New York (1989)). As shown in Scheme I, aryl amines are commonly synthesized by reduction of nitroaryls using a metal catalyst, such as Ni, Pd, or Pt, and H₂ or a hydride transfer agent, such as formate, cyclohexadiene, or a borohydride (Rylander. Hydrogenation Methods; Academic Press: London, UK (1985)). Nitroaryls may also be directly reduced using a strong hydride source, such as LiAlH₄ (Seyden-Penne. Reductions by the Alumino- and Borohydrides in Organic Synthesis; VCH Publishers: New York (1991)), or using a zero valent metal, such as Fe, Sn or Ca, often in acidic media. Many methods exist for the synthesis of nitroaryls (March. Advanced Organic Chemistry, 3rd Ed.; John Wiley: New York (1985). Larock. Comprehensive Organic Transformations; VCH Publishers: New York (1989)). 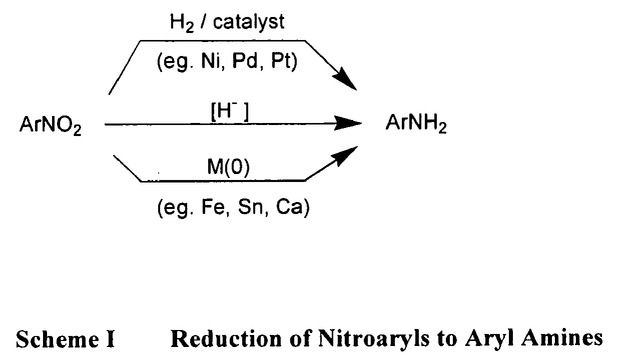 Nitroaryls are commonly formed by electrophilic aromatic nitration using HNO₃, or an alternative NO₂⁺ source. Nitroaryls may be further elaborated prior to reduction. Thus, nitroaryls substituted with 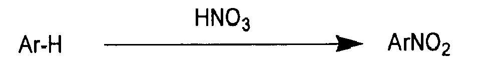 potential leaving groups (e.g. F, Cl, Br, etc.) may undergo substitution reactions on treatment with nucleophiles, such as thiolate (exemplified in Scheme II) or phenoxide. Nitroaryls may also undergo Ullman-type coupling reactions (Scheme II). 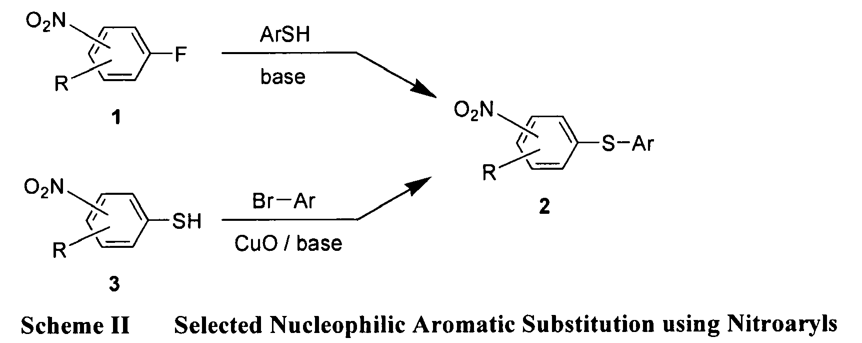 Nitroaryls may also undergo transition metal mediated cross coupling reactions. For example, nitroaryl electrophiles, such as nitroaryl bromides, iodides or triflates, undergo palladium mediated cross coupling reactions with aryl nucleophiles, such as arylboronic acids (Suzuki reactions, exemplified below), aryltins (Stille reactions) or arylzincs (Negishi reaction) to afford the biaryl (**5**). 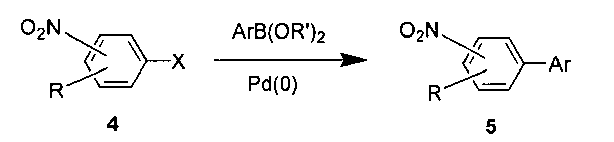 As shown in Scheme III, non-symmetrical urea formation may involve reaction of an aryl isocyanate (**14**) with an aryl amine (**13**). The heteroaryl isocyanate may be synthesized from a heteroaryl amine by treatment with phosgene or a phosgene equivalent, such as trichloromethyl chloroformate (diphosgene), bis(trichloromethyl) carbonate (triphosgene), or *N,N*'-carbonyldiimidazole (CDI). The isocyanate may also be derived from a heterocyclic carboxylic acid derivative, such as an ester, an acid halide or an anhydride by a Curtius-type rearrangement. Thus, reaction of acid derivative **16** with an azide source, followed by rearrangement affords the isocyanate. The corresponding carboxylic acid (**17**) may also be subjected to Curtius-type rearrangements using diphenylphosphoryl azide (DPPA) or a similar reagent.

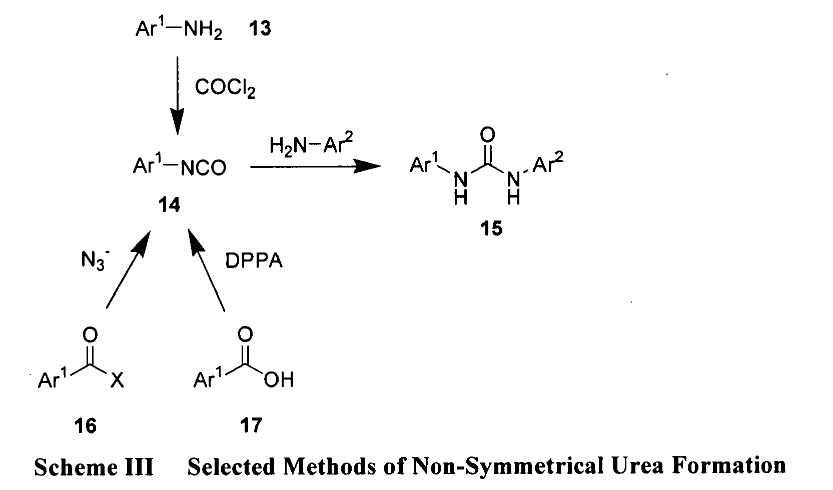

Finally, ureas may be further manipulated using methods familiar to those skilled in the art.

The compounds may be administered orally, topically, parenterally, by inhalation or spray or rectally in dosage unit formulations. The term 'administration by injection' includes intravenous, intramuscular, subcutaneous and parenteral injections, as well as use of infusion techniques. One or more compounds may be present in association with one or more non-toxic pharmaceutically acceptable carriers and if desired other active ingredients.

Compositions intended for oral use may be prepared according to any suitable method known to the art for the manufacture of pharmaceutical compositions. Such compositions may contain one or more agents selected from the group consisting of diluents, sweetening agents, flavoring agents, coloring agents and preserving agents in order to provide palatable preparations. Tablets contain the active ingredient in admixture with non-toxic pharmaceutically acceptable excipients which are suitable for the manufacture of tablets. These excipients may be, for example, inert diluents, such as calcium carbonate, sodium carbonate, lactose, calcium phosphate or sodium phosphate; granulating and disintegrating agents, for example, corn starch, or alginic acid; and binding agents, for example magnesium stearate, stearic acid or talc. The tablets may be uncoated or they may be coated by known techniques to delay disintegration and adsorption in the gastrointestinal tract and thereby provide a sustained action over a longer period. For example, a time delay material such as glyceryl monostearate or glyceryl distearate may be employed. These compounds may also be prepared in solid, rapidly released form.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Aqueous suspensions contain the active materials in admixture with excipients suitable for the manufacture of aqueous suspensions. Such excipients are suspending agents, for example sodium carboxymethylcellulose, methylcellulose, hydroxypropyl methylcellulose, sodium alginate, polyvinylpyrrolidone, gum tragacanth and gum acacia; dispersing or wetting agents may be a naturally occurring phosphatide, for example, lecithin, or condensation products or an alkylene oxide with fatty acids, for example polyoxyethylene stearate, or condensation products of ethylene oxide with long chain aliphatic alcohols, for example heptadecaethylene oxycetanol, or condensation products of ethylene oxide with partial esters derived from fatty acids and hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or n-propyl p-hydroxybenzoate, one or more coloring agents, one or more flavoring agents, and one or more sweetening agents, such as sucrose or saccharin.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example, sweetening, flavoring and coloring agents, may also be present.

The compounds may also be in the form of non-aqueous liquid formulations, e.g., oily suspensions which may be formulated by suspending the active ingredients in a vegetable oil, for example arachis oil, olive oil, sesame oil or peanut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide palatable oral preparations. These compositions may be preserved by the addition of an anti-oxidant such as ascorbic acid.

Pharmaceutical compositions of the invention may also be in the form of oil-in-water emulsions. The oily phase may be a vegetable oil, for example olive oil or arachis oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative and flavoring and coloring agents.

The compounds may also be administered in the form of suppositories for rectal administration of the drug. These compositions can be prepared by mixing the drug with a suitable non-irritating excipient which is solid at ordinary temperatures but liquid at the rectal temperature and will therefore melt in the rectum to release the drug. Such materials include cocoa butter and polyethylene glycols.

The compounds of this invention may also be administered parenterally, that is, subcutaneously, intravenously, intraocularly, intrasynovially, intramuscularly, or interperitoneally, as injectable dosages of the compound in a physiologically acceptable diluent with a pharmaceutical carrier which can be a sterile liquid or mixture of liquids such as water, saline, aqueous dextrose and related sugar solutions, an alcohol such as ethanol, isopropanol, or hexadecyl alcohol, glycols such as propylene glycol or polyethylene glycol, glycerol ketals such as 2,2-dimethyl-1,1-dioxolane-4-methanol, ethers such as poly(ethylene glycol) 400, an oil, a fatty acid, a fatty acid ester or, a fatty acid glyceride, or an acetylated fatty acid glyceride, with or without the addition of a pharmaceutically acceptable surfactant such as a soap or a detergent, suspending agent such as pectin, carbomers, methycellulose, hydroxypropylmethylcellulose, or carboxymethylcellulose, or emulsifying agent and other pharmaceutical adjuvants.

Illustrative of oils which can be used in the parenteral formulations of this invention are those of petroleum, animal, vegetable, or synthetic origin, for example, peanut oil, soybean oil, sesame oil, cottonseed oil, corn oil, olive oil, petrolatum and mineral oil. Suitable fatty acids include oleic acid, stearic acid, isostearic acid and myristic acid. Suitable fatty acid esters are, for example, ethyl oleate and isopropyl myristate. Suitable soaps include fatty acid alkali metal, ammonium, and triethanolamine salts and suitable detergents include cationic detergents, for example dimethyl dialkyl ammonium halides, alkyl pyridinium halides, and alkylamine acetates; anionic detergents, for example, alkyl, aryl, and olefin sulfonates, alkyl, olefin, ether, and monoglyceride sulfates, and sulfosuccinates; non-ionic detergents, for example, fatty amine oxides, fatty acid alkanolamides, and poly(oxyethylene-oxypropylene)s or ethylene oxide or propylene oxide copolymers; and amphoteric detergents, for example, alkyl-beta-aminopropionates, and 2-alkylimidazoline quarternary ammonium salts, as well as mixtures.

The parenteral compositions of this invention will typically contain from about 0.5% to about 25% by weight of the active ingredient in solution. Preservatives and buffers may also be used advantageously. In order to minimize or eliminate irritation at the site of injection, such compositions may contain a non-ionic surfactant having a hydrophile-lipophile balance (HLB) of from about 12 to about 17. The quantity of surfactant in such formulation ranges from about 5% to about 15% by weight. The surfactant can be a single component having the above HLB or can be a mixture of two or more components having the desired HLB.

Illustrative of surfactants used in parenteral formulations are the class of polyethylene sorbitan fatty acid esters, for example, sorbitan monooleate and the high molecular weight adducts of ethylene oxide with a hydrophobic base, formed by the condensation of propylene oxide with propylene glycol.

The pharmaceutical compositions may be in the form of sterile injectable aqueous suspensions. Such suspensions may be formulated according to known methods using suitable dispersing or wetting agents and suspending agents such as, for example, sodium carboxymethylcellulose, methylcellulose, hydroxypropylmethylcellulose, sodium alginate, polyvinylpyrrolidone, gum tragacanth and gum acacia; dispersing or wetting agents which may be a naturally occurring phosphatide such as lecithin, a condensation product of an alkylene oxide with a fatty acid, for example, polyoxyethylene stearate, a condensation product of ethylene oxide with a long chain aliphatic alcohol, for example, heptadeca-ethyleneoxycetanol, a condensation product of ethylene oxide with a partial ester derived form a fatty acid and a hexitol such as polyoxyethylene sorbitol monooleate, or a condensation product of an ethylene oxide with a partial ester derived from a fatty acid and a hexitol anhydride, for example polyoxyethylene sorbitan monooleate.

The sterile injectable preparation may also be a sterile injectable solution or suspension in a non-toxic parenterally acceptable diluent or solvent. Diluents and solvents that may be employed are, for example, water, Ringer's solution, isotonic sodium chloride solutions and isotonic glucose solutions. In addition, sterile fixed oils are conventionally employed as solvents or suspending media. For this purpose, any bland, fixed oil may be employed including synthetic mono- or diglycerides. In addition, fatty acids such as oleic acid can be used in the preparation of injectables.

Compounds of the invention may also be administrated transdermally using methods ("patches") known to those skilled in the art (see, for example: Chien; "Transdermal Controlled Systemic Medications"; Marcel Dekker, Inc.; 1987. Lipp et al. WO94/04157 3Mar94). Such transdermal patches may be used to provide continuous or discontinuous infusion of the compounds of the present invention in controlled amounts. The construction and use of transdermal patches for the delivery of pharmaceutical agents is well known in the art (see, e.g., US Patent No. 5,023,252, issued June 11, 1991, incorporated herein by reference). Such patches may be constructed for continuous, pulsatile, or on demand delivery of pharmaceutical agents. For example, a solution or suspension of a compound of Formula I in a suitable volatile solvent optionally containing penetration enhancing agents can be combined with additional additives known to those skilled in the art, such as matrix materials and bacteriocides. After sterilization, the resulting mixture can be formulated following known procedures into dosage forms. In addition, on treatment with emulsifying agents and water, a solution or suspension of a compound of Formula I may be formulated into a lotion or salve.

Suitable solvents for processing transdermal delivery systems are known to those skilled in the art, and include lower alcohols such as ethanol or isopropyl alcohol, lower ketones such as acetone, lower carboxylic acid esters such as ethyl acetate, polar ethers such as tetrahydrofuran, lower hydrocarbons such as hexane, cyclohexane or benzene, or halogenated hydrocarbons such as dichloromethane, chloroform, trichlorotrifluoroethane, or trichlorofluoroethane. Suitable solvents may also include mixtures of one or more materials selected from lower alcohols, lower ketones, lower carboxylic acid esters, polar ethers, lower hydrocarbons, halogenated hydrocarbons.

Suitable penetration enhancing materials for transdermal delivery system are known to those skilled in the art, and include, for example, monohydroxy or polyhydroxy alcohols such as ethanol, propylene glycol or benzyl alcohol, saturated or unsaturated C₈-C₁₈ fatty alcohols such as lauryl alcohol or cetyl alcohol, saturated or unsaturated C₈-C₁₈ fatty acids such as stearic acid, saturated or unsaturated fatty esters with up to 24 carbons such as methyl, ethyl, propyl, isopropyl, n-butyl, sec-butyl isobutyl tertbutyl or monoglycerin esters of acetic acid, capronic acid, lauric acid, myristinic acid, stearic acid, or palmitic acid, or diesters of saturated or unsaturated dicarboxylic acids with a total of up to 24 carbons such as diisopropyl adipate, diisobutyl adipate, diisopropyl sebacate, diisopropyl maleate, or diisopropyl fumarate. Additional penetration enhancing materials include phosphatidyl derivatives such as lecithin or cephalin, terpenes, amides, ketones, ureas and their derivatives, and ethers such as dimethyl isosorbid and diethyleneglycol monoethyl ether. Suitable penetration enhancing formulations may also include mixtures of one or more materials selected from monohydroxy or polyhydroxy alcohols, saturated or unsaturated C₈-C₁₈ fatty alcohols, saturated or unsaturated C₈-C₁₈ fatty acids, saturated or unsaturated fatty esters with up to 24 carbons, diesters of saturated or unsaturated discarboxylic acids with a total of up to 24 carbons, phosphatidyl derivatives, terpenes, amides, ketones, ureas and their derivatives, and ethers.

Suitable binding materials for transdermal delivery systems are known to those skilled in the art and include polyacrylates, silicones, polyurethanes, block polymers, styrenebutadiene copolymers, and natural and synthetic rubbers. Cellulose ethers, derivatized polyethylenes, and silicates may also be used as matrix components. Additional additives, such as viscous resins or oils may be added to increase the viscosity of the matrix.

Controlled release formulations for parenteral administration include liposomal, polymeric microsphere and polymeric gel formulations which are known in the art.

It may be desirable or necessary to introduce the pharmaceutical composition to the patient via a mechanical delivery device. The construction and use of mechanical delivery devices for the delivery of pharmaceutical agents is well known in the art. Direct techniques for, for example, administering a drug directly to the brain usually involve placement of a drug delivery catheter into the patient's ventricular system to bypass the blood-brain barrier. One such implantable delivery system, used for the transport of agents to specific anatomical regions of the body, is described in US Patent No. 5,011,472, issued April 30, 1991.

The compositions of the invention can also contain other conventional pharmaceutically acceptable compounding ingredients, generally referred to as carriers or diluents, as necessary or desired. Conventional procedures for preparing such compositions in appropriate dosage forms can be utilized. Such ingredients and procedures include those described in the following references, each of which is incorporated herein by reference: Powell, M.F. et al, "Compendium of Excipients for Parenteral Formulations" " PDA Journal of Pharmaceutical Science & Technology 1998, 52(5), 238-311; Strickley, R.G "Parenteral Formulations of Small Molecule Therapeutics Marketed in the United States (1999)-Part-1" PDA Journal of Pharmaceutical Science & Technology 1999, 53(6), 324-349; and Nema, S. et al, "Excipients and Their Use in Injectable Products" PDA Journal of Pharmaceutical Science & Technology 1997, 51(4), 166-171.

This invention also relates to administering pharmaceutical compositions containing one or more compounds of the present invention. These compositions can be utilized to achieve the desired pharmacological effect by administration to a patient in need thereof. A patient, for the purpose of this invention, is a mammal, including a human, in need of treatment for the particular condition or disease. Therefore, the present invention includes pharmaceutical compositions which are comprised of a pharmaceutically acceptable carrier and a pharmaceutically effective amount of a compound, or salt thereof, of the present invention. A pharmaceutically acceptable carrier is any carrier which is relatively non-toxic and innocuous to a patient at concentrations consistent with effective activity of the active ingredient so that any side effects ascribable to the carrier do not vitiate the beneficial effects of the active ingredient. A pharmaceutically effective amount of compound is that amount which produces a result or exerts an influence on the particular condition being treated. The compounds of the present invention can be administered with pharmaceutically-acceptable carriers well known in the art using any effective conventional dosage unit forms, including immediate, slow and timed release preparations, orally, parenterally, topically, nasally, ophthalmically, otically, sublingually, rectally, vaginally, and the like.

For oral administration, the compounds can be formulated into solid or liquid preparations such as capsules, pills, tablets, troches, lozenges, melts, powders, solutions, suspensions, or emulsions, and may be prepared according to methods known to the art for the manufacture of pharmaceutical compositions. The solid unit dosage forms can be a capsule which can be of the ordinary hard- or soft-shelled gelatin type containing, for example, surfactants, lubricants, and inert fillers such as lactose, sucrose, calcium phosphate, and corn starch.

In another embodiment, the compounds of this invention may be tableted with conventional tablet bases such as lactose, sucrose and cornstarch in combination with binders such as acacia, corn starch or gelatin, disintegrating agents intended to assist the break-up and dissolution of the tablet following administration such as potato starch, alginic acid, corn starch, and guar gum, gum tragacanth, acacia, lubricants intended to improve the flow of tablet granulation and to prevent the adhesion of tablet material to the surfaces of the tablet dies and punches, for example talc, stearic acid, or magnesium, calcium or zinc stearate, dyes, coloring agents, and flavoring agents such as peppermint, oil of wintergreen, or cherry flavoring, intended to enhance the aesthetic qualities of the tablets and make them more acceptable to the patient. Suitable excipients for use in oral liquid dosage forms include dicalcium phosphate and diluents such as water and alcohols, for example, ethanol, benzyl alcohol, and polyethylene alcohols, either with or without the addition of a pharmaceutically acceptable surfactant, suspending agent or emulsifying agent. Various other materials may be present as coatings or to otherwise modify the physical form of the dosage unit. For instance tablets, pills or capsules may be coated with shellac, sugar or both.

The compositions of the invention can also contain other conventional pharmaceutically acceptable compounding ingredients, generally referred to as carriers or diluents, as necessary or desired. Conventional procedures for preparing such compositions in appropriate dosage forms can be utilized. Such ingredients and procedures include those described in the following references, each of which is incorporated herein by reference: Powell, M.F. et al, "Compendium of Excipients for Parenteral Formulations" PDA Journal of Pharmaceutical Science & Technology 1998, 52(5), 238-311; Strickley, R.G "Parenteral Formulations of Small Molecule Therapeutics Marketed in the United States (1999)-Part-1" PDA Journal of Pharmaceutical Science & Technology 1999, 53(6), 324-349; and Nema, S. et al, "Excipients and Their Use in Injectable Products" PDA Journal of Pharmaceutical Science & Technology 1997, 51(4), 166-171.

Commonly used pharmaceutical ingredients which can be used as appropriate to formulate the composition for its intended route of administration include:
**acidifying agents** (examples include but are not limited to acetic acid, citric acid, fumaric acid, hydrochloric acid, nitric acid);
**alkalinizing agents** (examples include but are not limited to ammonia solution, ammonium carbonate, diethanolamine, monoethanolamine, potassium hydroxide, sodium borate, sodium carbonate, sodium hydroxide, triethanolamine, trolamine);
**adsorbents** (examples include but are not limited to powdered cellulose and activated charcoal);
**aerosol propellants** (examples include but are not limited to carbon dioxide, CCl₂F₂, F₂ClC-CClF₂ and CClF₃)
**air displacement agents** (examples include but are not limited to nitrogen and argon);
**antifungal preservatives** (examples include but are not limited to benzoic acid, butylparaben, ethylparaben, methylparaben, propylparaben, sodium benzoate);
**antimicrobial preservatives** (examples include but are not limited to benzalkonium chloride, benzethonium chloride, benzyl alcohol, cetylpyridinium chloride, chlorobutanol, phenol, phenylethyl alcohol, phenylmercuric nitrate and thimerosal);
**antioxidants** (examples include but are not limited to ascorbic acid, ascorbyl palmitate, butylated hydroxyanisole, butylated hydroxytoluene, hypophosphorus acid, monothioglycerol, propyl gallate, sodium ascorbate, sodium bisulfite, sodium formaldehyde sulfoxylate, sodium metabisulfite);
**binding materials** (examples include but are not limited to block polymers, natural and synthetic rubber, polyacrylates, polyurethanes, silicones, polysiloxanes and styrene-butadiene copolymers);
**buffering agents** (examples include but are not limited to potassium metaphosphate, dipotassium phosphate, sodium acetate, sodium citrate anhydrous and sodium citrate dihydrate)
**carrying agents** (examples include but are not limited to acacia syrup, aromatic syrup, aromatic elixir, cherry syrup, cocoa syrup, orange syrup, syrup, corn oil, mineral oil, peanut oil, sesame oil, bacteriostatic sodium chloride injection and bacteriostatic water for injection)
**chelating agents** (examples include but are not limited to edetate disodium and edetic acid)
**colorants** (examples include but are not limited to FD&C Red No. 3, FD&C Red No. 20, FD&C Yellow No. 6, FD&C Blue No. 2, D&C Green No. 5, D&C Orange No. 5, D&C Red No. 8, caramel and ferric oxide red);
**clarifying agents** (examples include but are not limited to bentonite);
**emulsifying agents** (examples include but are not limited to acacia, cetomacrogol, cetyl alcohol, glyceryl monostearate, lecithin, sorbitan monooleate, polyoxyethylene 50 monostearate);
**encapsulating agents** (examples include but are not limited to gelatin and cellulose acetate phthalate)
**flavorants** (examples include but are not limited to anise oil, cinnamon oil, cocoa, menthol, orange oil, peppermint oil and vanillin);
**humectants** (examples include but are not limited to glycerol, propylene glycol and sorbitol);
**levigating agents** (examples include but are not limited to mineral oil and glycerin);
**oils** (examples include but are not limited to arachis oil, mineral oil, olive oil, peanut oil, sesame oil and vegetable oil);
**ointment bases** (examples include but are not limited to lanolin, hydrophilic ointment, polyethylene glycol ointment, petrolatum, hydrophilic petrolatum, white ointment, yellow ointment, and rose water ointment);
**penetration enhancers (transdermal delivery)** (examples include but are not limited to monohydroxy or polyhydroxy alcohols, mono-or polyvalent alcohols, saturated or unsaturated fatty alcohols, saturated or unsaturated fatty esters, saturated or unsaturated dicarboxylic acids, essential oils, phosphatidyl derivatives, cephalin, terpenes, amides, ethers, ketones and ureas)
**plasticizers** (examples include but are not limited to diethyl phthalate and glycerol);
**solvents** (examples include but are not limited to ethanol, corn oil, cottonseed oil, glycerol, isopropanol, mineral oil, oleic acid, peanut oil, purified water, water for injection, sterile water for injection and sterile water for irrigation);
**stiffening agents** (examples include but are not limited to cetyl alcohol, cetyl esters wax, microcrystalline wax, paraffin, stearyl alcohol, white wax and yellow wax);
**suppository bases** (examples include but are not limited to cocoa butter and polyethylene glycols (mixtures));
**surfactants** (examples include but are not limited to benzalkonium chloride, nonoxynol 10, oxtoxynol 9, polysorbate 80, sodium lauryl sulfate and sorbitan monopalmitate);
**suspending agents** (examples include but are not limited to agar, bentonite, carbomers, carboxymethylcellulose sodium, hydroxyethyl cellulose, hydroxypropyl cellulose, hydroxypropyl methylcellulose, kaolin, methylcellulose, tragacanth and veegum);
**sweetening agents** (examples include but are not limited to aspartame, dextrose, glycerol, mannitol, propylene glycol, saccharin sodium, sorbitol and sucrose);
**tablet anti-adherents (**examples include but are not limited to magnesium stearate and talc);
**tablet binders** (examples include but are not limited to acacia, alginic acid, carboxymethylcellulose sodium, compressible sugar, ethylcellulose, gelatin, liquid glucose, methylcellulose, non-crosslinked polyvinyl pyrrolidone, and pregelatinized starch);
**tablet and capsule diluents** (examples include but are not limited to dibasic calcium phosphate, kaolin, lactose, mannitol, microcrystalline cellulose, powdered cellulose, precipitated calcium carbonate, sodium carbonate, sodium phosphate, sorbitol and starch);
**tablet coating agents** (examples include but are not limited to liquid glucose, hydroxyethyl cellulose, hydroxypropyl cellulose, hydroxypropyl methylcellulose, methylcellulose, ethylcellulose, cellulose acetate phthalate and shellac);
**tablet direct compression excipients** (examples include but are not limited to dibasic calcium phosphate);
**tablet disintegrants** (examples include but are not limited to alginic acid, carboxymethylcellulose calcium, microcrystalline cellulose, polacrillin potassium, cross-linked polyvinylpyrrolidone, sodium alginate, sodium starch glycollate and starch);
**tablet glidants** (examples include but are not limited to colloidal silica, corn starch and talc);
**tablet lubricants** (examples include but are not limited to calcium stearate, magnesium stearate, mineral oil, stearic acid and zinc stearate);
**tablet/capsule opaquants** (examples include but are not limited to titanium dioxide);
**tablet polishing agents** (examples include but are not limited to carnauba wax and white wax);
**thickening agents** (examples include but are not limited to beeswax, cetyl alcohol and paraffin);
**tonicity agents** (examples include but are not limited to dextrose and sodium chloride);
**viscosity increasing agents** (examples include but are not limited to alginic acid, bentonite, carbomers, carboxymethylcellulose sodium, methylcellulose, polyvinyl pyrrolidone, sodium alginate and tragacanth); and
**wetting agents** (examples include but are not limited to heptadecaethylene oxycetanol, lecithins, sorbitol monooleate, polyoxyethylene sorbitol monooleate, and polyoxyethylene stearate).

The total amount of the active ingredient to be administered will generally range from about 0.001 mg/kg to about 200 mg/kg, and preferably from about 0.01 mg/kg to about 20 mg/kg body weight per day. A unit dosage may contain from about 0.5 mg to about 1500 mg of active ingredient, and can be administered one or more times per day. For all regimens of use disclosed herein for compounds of Formula I, the daily oral dosage regimen will preferably be from 0.01 to 200 mg/Kg of total body weight. The daily dosage for administration by injection, including intravenous, intramuscular, subcutaneous and parenteral injections, and use of infusion techniques will preferably be from 0.01 to 200 mg/Kg of total body weight. The daily rectal dosage regime will preferably be from 0.01 to 200 mg/Kg of total body weight. The daily vaginal dosage regime will preferably be from 0.01 to 200 mg/Kg of total body weight. The daily topical dosage regime will preferably be from 0.1 to 200 mg administered between one to four times daily. The transdermal concentration will preferably be that required to maintain a daily dose of from 0.01 to 200 mg/Kg. The daily inhalation dosage regime will preferably be from 0.01 to 100 mg/Kg of total body weight. These dosages regimes can be achieved with multiple dosages within a single day or extended dosages, such as those given on a weekly or monthly basis.

Based upon standard laboratory techniques known to evaluate compounds useful for the treatment of hyper-proliferative disorders, by standard toxicity tests and by standard pharmacological assays for the determination of treatment of the conditions identified above in mammals, and by comparison of these results with the results of known medicaments that are used to treat these conditions, the effective dosage of the compounds of this invention can readily be determined for treatment of each desired indication. The amount of the active ingredient to be administered in the treatment of one of these conditions can vary widely according to such considerations as the particular compound and dosage unit employed, the mode of administration, the period of treatment, the age and gender of the patient treated, and the nature and extent of the condition treated.

It will be appreciated by those skilled in the art that the particular method of administration will depend on a variety of factors, all of which are considered routinely when administering therapeutics. It will also be appreciated by one skilled in the art that the specific dose level for a given patient depends on a variety of factors, including specific activity of the compound administered, age, body weight, health, sex, diet, time and route of administration, rate of excretion, etc. It will be further appreciated by one skilled in the art that the optimal course of treatment, i.e., the mode of treatment and the daily number of doses of a compound of Formula I or a pharmaceutically acceptable salt thereof given for a defined number of days, can be ascertained by those skilled in the art using conventional treatment tests.

It will be understood, however, that the specific dose level for any particular patient will depend upon a variety of factors, including the activity of the specific compound employed, the age, body weight, general health, sex, diet, time of administration, route of administration, and rate of excretion, drug combination and the severity of the condition undergoing therapy.

It will be further appreciated by one skilled in the art that the optimal course of treatment, i.e., the mode of treatment and the daily number of doses of a compound of this invention given for a defined number of days, can be ascertained by those skilled in the art using conventional treatment tests.

Specific preparations of the compounds of this invention are already described in the patent literature, and can be adapted to the compounds of the present invention. For example, Riedl, B., et al., "O-Carboxy Aryl Substituted Diphenyl Ureas as raf Kinase Inhibitors" *PCT Int. Appl.,* WO 00 42012, Riedl, B., et al., "O-Carboxy Aryl Substituted Diphenyl Ureas as p38 Kinase Inhibitors" *PCT Int. Appl.,* WO 00 41698, incorporated herein by reference.

Pharmaceutical compositions according to the present invention can be illustrated as follows:
**Sterile IV Solution**: A 5 mg/ml solution of the desired compound of this invention is made using sterile, injectable water, and the pH is adjusted if necessary. The solution is diluted for administration to 1 - 2 mg/ml with sterile 5% dextrose and is administered as an IV infusion over 60 minutes.
**Lyophilized powder for IV administration**: A sterile preparation can be prepared with (i) 100 - 1000 mg of the desired compound of this invention as a lyophilized powder, (ii) 32- 327 mg/ml sodium citrate, and (iii) 300 - 3000 mg Dextran 40. The formulation is reconstituted with sterile, injectable saline or dextrose 5% to a concentration of 10 to 20 mg/ml, which is further diluted with saline or dextrose 5% to 0.2 - 0.4 mg/ml, and is administered either IV bolus or by IV infusion over 15 - 60 minutes.
**Intramuscular suspension**: The following solution or suspension can be prepared, for intramuscular injection:
   50 mg/ml of the desired, water-insoluble compound of this invention
   5 mg/ml sodium carboxymethylcellulose
   4 mg/ml TWEEN 80
   9 mg/ml sodium chloride
   9 mg/ml benzyl alcohol
**Hard Shell Capsules:** A large number of unit capsules are prepared by filling standard two-piece hard galantine capsules each with 100 mg of powdered active ingredient, 150 mg of lactose, 50 mg of cellulose and 6 mg of magnesium stearate.
**Soft Gelatin Capsules:** A mixture of active ingredient in a digestible oil such as soybean oil, cottonseed oil or olive oil is prepared and injected by means of a positive displacement pump into molten gelatin to form soft gelatin capsules containing 100 mg of the active ingredient. The capsules are washed and dried. The active ingredient can be dissolved in a mixture of polyethylene glycol, glycerin and sorbitol to prepare a water miscible medicine mix.
**Tablets:** A large number of tablets are prepared by conventional procedures so that the dosage unit was 100 mg of active ingredient, 0.2 mg. of colloidal silicon dioxide, 5 mg of magnesium stearate, 275 mg of microcrystalline cellulose, 11 mg. of starch, and 98.8 mg of lactose. Appropriate aqueous and non-aqueous coatings may be applied to increase palatability, improve elegance and stability or delay absorption.
Immediate Release Tablets/Capsules: These are solid oral dosage forms made by conventional and novel processes. These units are taken orally without water for immediate dissolution and delivery of the medication. The active ingredient is mixed in a liquid containing ingredient such as sugar, gelatin, pectin and sweeteners. These liquids are solidified into solid tablets or caplets by freeze drying and solid-state extraction techniques. The drug compounds may be compressed with viscoelastic and thermoelastic sugars and polymers or effervescent components to produce porous matrices intended for immediate release, without the need of water.

The compopund N-[4-chloro-3-(trifluoromethyl)phenyl]-N'-{4-[2-carbamoyl-1-oxo-(4-pyridyloxy)]phenyl} urea, as referred to herein, can be prepared by the following multistep procedure:

### Step 1: Preparation of 4-chloro-2-pyridinecarboxamide

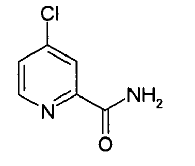

To a stirred mixture of methyl 4-chloro-2-pyridinecarboxylate hydrochloride (1.0 g, 4.81 mmol) dissolved in conc. aqueous ammonia (32 mL) is added ammonium chloride (96.2 mg, 1.8 mmol, 0.37 equiv.), and the heterogeneous reaction mixture is stirred at ambient temperature for 16h. The reaction mixture is poured into EtOAc (500 mL) and water (300 mL). The organic layer is washed with water (2 x 300 mL) and a saturated NaCl solution (1 x 300 mL), dried (MgSO₄), concentrated in *vacuo* to give 4-chloro-2-pyridinecarboxamide as a beige solid (604.3 mg, 80.3%): TLC (50% EtOAc / hexane) R_{f} 0.20; ¹H-NMR (DMSO-d₆) δ 8.61 (d, J = 5.4 Hz, 1H), 8.20 (broad s, 1H), 8.02 (d, J = 1.8 Hz, 1H), 7.81 (broad s, 1H), 7.76 to 7.73 (m, 1H).

### Step 2: Preparation of 4-(4-aminophenoxy)-2-pyridinecarboxamide

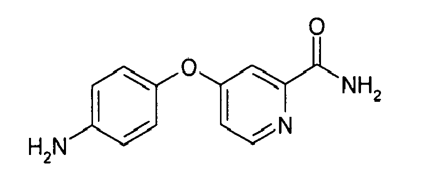

To 4-aminophenol (418 mg, 3.83 mmol) in anh DMF(7.7 mL) is added potassium *tert*-butoxide (447 mg, 3.98 mmol, 1.04 equiv.) in one portion. The reaction mixture is stirred at room temperature for 2 h, and a solution of 4-chloro-2-pyridinecarboxamide (600 mg, 3.83 mmol, 1.0 equiv.) in anh DMF (4 mL) is then added. The reaction mixture is stirred at 80 °C for 3 days and poured into a mixture of EtOAc and a saturated NaCl solution. The organic layer is sequentially washed with a saturated NH₄Cl solution then a saturated NaCl solution, dried (MgSO₄), and concentrated under reduced pressure. The crude product is purified using MPLC chromatography (Biotage^{®}; gradient from 100% EtOAc to followed by 10% MeOH /50% EtOAc / 40% hexane) to give the 4-chloro-5-trifluoromethylaniline as a brown solid (510 mg, 58%). ¹H-NMR (DMSO-d₆) δ 8.43 (d, J = 5.7 Hz, 1H), 8.07 (br s, 1H), 7.66 (br s, 1H), 7.31 (d, J = 2.7 Hz, 1H), 7.07 (dd, J = 5.7 Hz, 2.7 Hz, 1H), 6.85 (d, J = 9.0 Hz, 2 H), 6.62 (d, J = 8.7 Hz, 2H), 5.17 (broad s, 2H); HPLC EI-MS *m*/*z* 230 ((M+H)⁺.

### Step 3: Preparation of N-[4-chloro-3-(trifluoromethyl)phenyl]-N'-{4-[2-carbamoyl-(4-pyridyloxy)]phenyl} urea

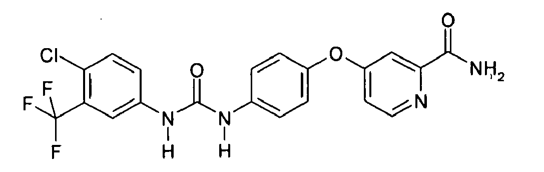

A mixture of 4-chloro-5-trifluoromethylaniline (451 mg, 2.31 mmol, 1.1 equiv.) and 1,1'-carbonyl diimidazole (419 mg, 2.54 mmol, 1.2 equiv.) in anh dichloroethane (5.5 mL) is stirred under argon at 65 °C for 16 h. Once cooled to room temperature, a solution of 4-(4-aminophenoxy)-2-pyridinecarboxamide (480 mg, 2.09 mmol) in anh THF (4.0 mL) is added, and the reaction mixture is stirred at 60°C for 4 h. The reaction mixture is poured into EtOAc, and the organic layer is washed with water (2x) and a saturated NaCl solution (Ix), dried (MgSO₄), filtered, and evaporated in *vacuo.* Purification using MPLC chromatography (Biotage^{®}; gradient from 100% EtOAc to 2% MeOH / EtOAc) gave N-[4-chloro-3-(trifluoromethyl)phenyl]-N'-{4-[2-carbamoyl-(4-pyridyloxy)]phenyl} urea as a white solid (770 mg, 82%): TLC (EtOAc) R_{f} 0.11, 100% ethyl acetate ¹H-NMR (DMSO-d₆) δ 9.21 (s, 1H), 8.99 (s, 1H), 8.50 (d, J = 5.6 Hz, 1H), 8.11 (s, 1H), 8.10 (s, 1H), 7.69 (broad s, 1H), 7.64 (dd, J = 8.2 Hz, 2.1 Hz, 1H), 7.61 (s, 1H), 7.59 (d, J = 8.8 Hz, 2H), 7.39 (d, J = 2.5 Hz, 1H), 7.15 (d, J = 8.9 Hz, 2H), 7.14 (m, 1H); MS LC-MS (MH⁺ = 451). Anal. calcd for C₂₀H₁₄ClF₃N₄O₃: C 53.29% H 3.13% N 12.43%. Found: C 53.33% H 3.21% N 12.60%.

Other methods of preparing N-[4-chloro-3-(trifluoromethyl)phenyl]-N'-{4-[2-carbamoyl-(4-pyridyloxy)]phenyl} urea are described in Bankston et al. "A Scaleable Synthesis of BAY 43-9006: A Potent Raf Kinase Inhibitor for the Treatment of Cancer" Org. Proc. Res. Dev. 2002, 6(6), 777-781, and WO 00/42012 and WO 00/41698.

An example of the preparation of "Sorafenib,"4-{4-[({[4-Chloro-3-(trifluoromethyl)phenyl]amino}carbonyl)amino]phenoxy}-N-methylpyridine-2-carboxamide tosylate, in the polymorph II is s follows:
903 g of4-{4-[({[4-chloro-3-(trifluoromethyl)phenyl]amino}carbonyl)amino]phenoxy}-N-methylpyridine-2-carboxamide, prepared as described above, are initially charged in 2700 ml of ethanol. 451.7 g of p-toluenesulfonic acid monohydrate are dissolved in 1340 g of ethanol and added dropwise at room temperature. The suspension is stirred at room temperature for 1 hour, then filtered off with suction, and the residue is washed three times with 830 ml each time of ethanol. The drying is effected at 50°C under reduced pressure with supply of air. 1129.6 g of the title compound in the polymorph II are obtained.

An example of the preparation of "Sorafenib," 4-{4-[({[4-chloro-3-(trifluoromethyl)phenyl]amino}carbonyl)-amino]phenoxy}-N-methylpyridine-2-carboxamide tosylate, in the polymorph I is as follows:
Heating 5mg of "Sorafenib,"[tosylate salt of 4-{4-[({[4-chloro-3-(trifluoromethyl)phenyl]amino}carbonyl)amino]-phenoxy}-N-methylpyridine-2-carboxamide] in the polymorph II to 200°C at a heating rate of 20°C/min and subsequently cooling to room temperature at a cooling rate of 2°C/min. The sample is tested thermoanalytically (DSC) and corresponds to the title compound in the polymorph I.

Methods for preparing the compounds of this invention are also described in the following U.S. applications:.
09/425,228, filed October 22, 1999;
09/722,418 filed November 28, 2000
09/758,547, filed January 12, 2001;
09/838,285, filed April 20, 2001;
09/838,286, filed April 20, 2001; and

The entire disclosure of all applications, patents and publications cited above and below including copending application Serial No. 10/308,187 filed December 12, 2002 and Serial No. 10/848,567 filed May 19, 2004 and International application Serial No. PCT/US/04/15655, filed May 19, 2004., are hereby incorporated by reference.

The compounds can be produced from known compounds (or from starting materials which, in turn, can be produced from known compounds), e.g., through the general preparative methods shown below. The activity of a given compound to inhibit raf kinase can be routinely assayed, e.g., according to procedures disclosed below. The following examples are for illustrative purposes only and are not intended, nor should they be construed to limit the invention in any way.

### EXAMPLES

All reactions were performed in flame-dried or oven-dried glassware under a positive pressure of dry argon or dry nitrogen, and were stirred magnetically unless otherwise indicated. Sensitive liquids and solutions were transferred via syringe or cannula, and introduced into reaction vessels through rubber septa. Unless otherwise stated, the term 'concentration under reduced pressure' refers to use of a Buchi rotary evaporator at approximately 15 mmHg. Unless otherwise stated, the term 'under high vacuum' refers to a vacuum of 0.4 - 1.0 mmHg.

All temperatures are reported uncorrected in degrees Celsius (°C). Unless otherwise indicated, all parts and percentages are by weight.

Commercial grade reagents and solvents were used without further purification. *N-*cyclohexyl-*N'*-(methylpolystyrene)carbodiimide was purchased from Calbiochem-Novabiochem Corp. 3-*tert*-Butylaniline, 5-*tert*-butyl-2-methoxyaniline, 4-bromo-3-(trifluoromethyl)aniline, 4-chloro-3-(trifluoromethyl)aniline 2-methoxy-5-(trifluoromethyl)aniline, 4-*tert*-butyl-2-nitroaniline, 3-amino-2-naphthol, ethyl 4-isocyanatobenzoate, *N*-acetyl-4-chloro-2-methoxy-5-(trifluoromethyl)aniline and 4-chloro-3-(trifluoromethyl)phenyl isocyanate were purchased and used without further purification. Syntheses of 3-amino-2-methoxyquinoline (E. Cho et al. WO 98/00402; A. Cordi et al. EP 542,609; IBID Bioorg. Med. Chem.. 3, 1995, 129), 4-(3-carbamoylphenoxy)-1-nitrobenzene (K. Ikawa Yakugaku Zasshi 79, 1959, 760; Chem. Abstr. 53, 1959, 12761b), 3-*tert*-butylphenyl isocyanate (O. Rohr et al. DE 2,436,108) and 2-methoxy-5-(trifluoromethyl)phenyl isocyanate (K. Inukai et al. JP 42,025,067; IBID Kogyo Kagaku Zasshi 70, 1967, 491) have previously been described.

Thin-layer chromatography (TLC) was performed using Whatman^{®} pre-coated glass-backed silica gel 60A F-254 250 µm plates. Visualization of plates was effected by one or more of the following techniques: (a) ultraviolet illumination, (b) exposure to iodine vapor, (c) immersion of the plate in a 10% solution of phosphomolybdic acid in ethanol followed by heating, (d) immersion of the plate in a cerium sulfate solution followed by heating, and/or (e) immersion of the plate in an acidic ethanol solution of 2,4-dinitrophenylhydrazine followed by heating. Column chromatography (flash chromatography) was performed using 230-400 mesh EM Science^{®} silica gel.

Melting points (mp) were determined using a Thomas-Hoover melting point apparatus or a Mettler FP66 automated melting point apparatus and are uncorrected. Fourier transform infrared spectra were obtained using a Mattson 4020 Galaxy Series spectrophotometer. Proton (¹H) nuclear magnetic resonance (NMR) spectra were measured with a General Electric GN-Omega 300 (300 MHz) spectrometer with either Me₄Si (δ 0.00) or residual protonated solvent (CHCl₃ δ 7.26; MeOH δ 3.30; DMSO δ 2.49) as standard. Carbon (¹³C) NMR spectra were measured with a General Electric GN-Omega 300 (75 MHz) spectrometer with solvent (CDCl₃ δ 77.0; MeOD-d₃; δ 49.0; DMSO-d₆ δ 39.5) as standard. Low-resolution mass spectra (MS) and high resolution mass spectra (HRMS) were either obtained as electron impact (EI) mass spectra or as fast atom bombardment (FAB) mass spectra. Electron impact mass spectra (EI-MS) were obtained with a Hewlett Packard 5989A mass spectrometer equipped with a Vacumetrics Desorption Chemical Ionization Probe for sample introduction. The ion source was maintained at 250 °C. Electron impact ionization was performed with electron energy of 70 eV and a trap current of 300 µA. Liquid-cesium secondary ion mass spectra (FAB-MS), an updated version of fast atom bombardment were obtained using a Kratos Concept 1-H spectrometer. Chemical ionization mass spectra (CI-MS) were obtained using a Hewlett Packard MS-Engine (5989A) with methane or ammonia as the reagent gas (1x10⁻⁴ torr to 2.5x10⁻⁴ torr). The direct insertion desorption chemical ionization (DCI) probe (Vaccumetrics, Inc.) was ramped from 0-1.5 amps in 10 sec and held at 10 amps until all traces of the sample disappeared (∼1-2 min). Spectra were scanned from 50-800 amu at 2 sec per scan. HPLC - electrospray mass spectra (HPLC ES-MS) were obtained using a Hewlett-Packard 1100 HPLC equipped with a quaternary pump, a variable wavelength detector, a C-18 column, and a Finnigan LCQ ion trap mass spectrometer with electrospray ionization. Spectra were scanned from 120-800 amu using a variable ion time according to the number of ions in the source. Gas chromatography - ion selective mass spectra (GC-MS) were obtained with a Hewlett Packard 5890 gas chromatograph equipped with an HP-1 methyl silicone column (0.33 mM coating; 25 m x 0.2 mm) and a Hewlett Packard 5971 Mass Selective Detector (ionization energy 70 eV). Elemental analyses are conducted by Robertson Microlit Labs, Madison NJ.

All compounds displayed NMR spectra, LRMS and either elemental analysis or HRMS consistent with assigned structures.

### List of Abbreviations and Acronyms:

- AcOH: acetic acid
- anh: anhydrous
- atm: atmosphere(s)
- BOC: *tert*-butoxycarbonyl
- CDI: 1,1'-carbonyl diimidazole
- conc: concentrated
- d: day(s)
- dec: decomposition
- DMAC: *N,N*-dimethylacetamide
- DMPU: 1,3-dimethyl-3,4,5,6-tetrahydro-2(1H)-pyrimidinone
- DMF: *N,N*-dimethylformamide
- DMSO: dimethylsulfoxide
- DPPA: diphenylphosphoryl azide
- EDCI: 1-(3-dimethylaminopropyl)-3-ethylcarbodiimide
- EtOAc: ethyl acetate
- EtOH: ethanol (100%)
- Et₂O: diethyl ether
- Et₃N: triethylamine
- h: hour(s)

- HOBT: 1-hydroxybenzotriazole
- *m*-CPBA: 3-chloroperoxybenzoic acid
- MeOH: methanol
- pet. ether: petroleum ether (boiling range 30-60 °C)
- temp.: temperature
- THF: tetrahydrofuran
- TFA: trifluoroAcOH
- Tf: trifluoromethanesulfonyl

**The following general methods are described in copending application serial number** 09/948,915, filed September 10, 2001**, and are hereby incorporated by reference.**
A. General Methods for Synthesis of Substituted Anilines, pages 18-43
B. Synthesis of Urea Precursors, page 43,
C. Methods of Urea Formation, pages 44-51 and
D. Interconversion of Ureas, pages 52-56.

### EXAMPLE A

### N-[4-chloro-3-(trifluoromethyl)phenyl]-N'-{4-[2-carbamoyl-(4-yridyloxy)]phenyl} urea

### Step 1: Preparation of 4-chloro-2-pyridinecarboxamide

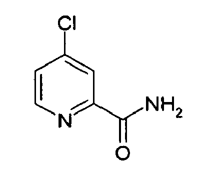

To a stirred mixture of methyl 4-chloro-2-pyridinecarboxylate hydrochloride (1.0 g, 4.81 mmol) dissolved in conc. aqueous ammonia (32 mL) was added ammonium chloride (96.2 mg, 1.8 mmol, 0.37 equiv.), and the heterogeneous reaction mixture was stirred at ambient temperature for 16h. The reaction mixture was poured into EtOAc (500 mL) and water (300 mL). The organic layer was washed with water (2 x 300 mL) and a saturated NaCl solution (1 x 300 mL), dried (MgSO₄), concentrated in *vacuo* to give 4-chloro-2-pyridinecarboxamide as a beige solid (604.3 mg, 80.3%): TLC (50% EtOAc / hexane) R_{f} 0.20; ¹H-NMR (DMSO-d₆) δ 8.61 (d, J = 5.4 Hz, 1H), 8.20 (broad s, 1H), 8.02 (d, J = 1.8 Hz, 1H), 7.81 (broad s, 1H), 7.76 to 7.73 (m, 1H).

### Step 2: Preparation of 4-(4-aminophenoxy)-2-pyridinecarboxamide

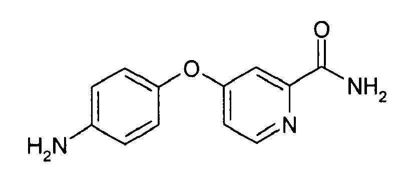

To 4-aminophenol (418 mg, 3.83 mmol) in anh DMF(7.7 mL) was added potassium *tert-butoxide* (447 mg, 3.98 mmol, 1.04 equiv.) in one portion. The reaction mixture was stirred at room temperature for 2 h, and a solution of 4-chloro-2-pyridinecarboxamide (600 mg, 3.83 mmol, 1.0 equiv.) in anh DMF (4 mL) was then added. The reaction mixture was stirred at 80 °C for 3 days and poured into a mixture of EtOAc and a saturated NaCl solution. The organic layer was sequentially washed with a saturated NH₄Cl solution then a saturated NaCl solution, dried (MgSO₄), and concentrated under reduced pressure. The crude product was purified using MPLC chromatography (Biotage^{®}; gradient from 100% EtOAc to followed by 10% MeOH /50% EtOAc / 40% hexane) to give the 4-chloro-5-trifluoromethylaniline as a brown solid (510 mg, 58%). ¹H-NMR (DMSO-d₆) δ 8.43 (d, J = 5.7 Hz, 1H), 8.07 (br s, 1H), 7.66 (br s, 1H), 7.31 (d, J = 2.7 Hz, 1H), 7.07 (dd, J = 5.7 Hz, 2.7 Hz, 1H), 6.85 (d, J = 9.0 Hz, 2 H), 6.62 (d, J = 8.7 Hz, 2H), 5.17 (broad s, 2H); HPLC EI-MS *m*/*z* 230 ((M+H)⁺.

### Step 3: Preparation of N-[4-chloro-3-(trifluoromethyl)phenyl]-N'-{4-[2-carbamoyl-(4-pyridyloxy)]phenyl} urea

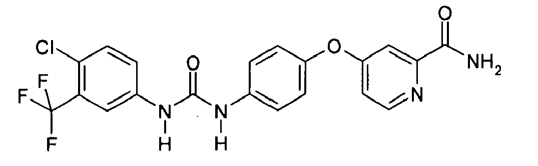

A mixture of 4-chloro-5-trifluoromethylaniline (451 mg, 2.31 mmol, 1.1 equiv.) and 1,1'-carbonyl diimidazole (419 mg, 2.54 mmol, 1.2 equiv.) in anh dichloroethane (5.5 mL) was stirred under argon at 65 °C for 16 h. Once cooled to room temperature, a solution of 4-(4-aminophenoxy)-2-pyridinecarboxamide (480 mg, 2.09 mmol) in anh THF (4.0 mL) was added, and the reaction mixture was stirred at 60°C for 4 h. The reaction mixture was poured into EtOAc, and the organic layer was washed with water (2x) and a saturated NaCl solution (1x), dried (MgSO₄), filtered, and evaporated in *vacuo.* Purification using MPLC chromatography (Biotage^{®}; gradient from 100% EtOAc to 2% MeOH / EtOAc) gave N-[4-chloro-3-(trifluoromethyl)phenyl]-N'-{4-[2-carbamoyl-(4-pyridyloxy)]phenyl} urea as a white solid (770 mg, 82%): TLC (EtOAc) R_{f} 0.11, 100% ethyl acetate ¹H-NMR (DMSO-d₆) δ 9.21 (s, 1H), 8.99 (s, 1H), 8.50 (d, J = 5.6 Hz, 1H), 8.11 (s, 1H), 8.10 (s, 1H), 7.69 (broad s, 1H), 7.64 (dd, J = 8.2 Hz, 2.1 Hz, 1H), 7.61 (s, 1H), 7.59 (d, J = 8.8 Hz, 2H), 7.39 (d, J = 2.5 Hz, 1H), 7.15 (d, J = 8.9 Hz, 2H), 7.14 (m, 1H); MS LC-MS (MH⁺ = 451). Anal. calcd for C₂₀H₁₄ClF₃N₄O₃: C 53.29% H 3.13% N 12.43%. Found: C 53.33% H 3.21% N 12.60%.

### Example B

### N-[4-chloro-3-(trifluoromethyl)phenyl]-N'-{4-[2-N-methylcarbamoyl-4-pyridyloxy]phenyl} urea

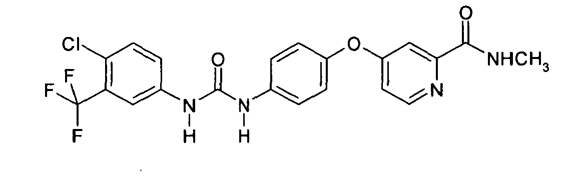

Step 1: 4-Chloro-*N*-methyl-2-pyridinecarboxamide is first synthesized from 4-chloropyridine-2-carbonyl chloride by adding 4-chloropyridine-2-carbonyl chloride HCl salt (7.0 g, 32.95 mmol) in portions to a mixture of a 2.0 M methylamine solution in THF (100 mL) and MeOH (20 mL) at 0 °C. The resulting mixture is stored at 3 °C for 4 h, then concentrated under reduced pressure. The resulting nearly dry solids are suspended in EtOAc (100 mL) and filtered. The filtrate is washed with a saturated NaCl solution (2 x 100 mL), dried (Na₂SO₄) and concentrated under reduced pressure to provide 4-chloro-N-methyl-2-pyridinecarboxamide as a yellow, crystalline solid.
Step 2: A solution of 4-aminophenol (9.60 g, 88.0 mmol) in anh. DMF (150 mL) is treated with potassium *tert-*butoxide (10.29 g, 91.7 mmol), and the reddish-brown mixture is stirred at room temp. for 2 h. The contents are treated with 4-chloro-N-methyl-2-pyridinecarboxamide (15.0 g, 87.9 mmol) from Step 1 and K₂CO₃ (6.50 g, 47.0 mmol) and then heated at 80 °C for 8 h. The mixture is cooled to room temp. and separated between EtOAc (500 mL) and a saturated NaCl solution (500 mL). The aqueous phase is back-extracted with EtOAc (300 mL). The combined organic layers are washed with a saturated NaCl solution (4 x 1000 mL), dried (Na₂SO₄) and concentrated under reduced pressure. The resulting solids are dried under reduced pressure at 35 °C for 3 h to afford 4-(2-(*N*-methylcarbamoyl)-4-pyridyloxy)aniline as a light-brown solid. ¹H-NMR (DMSO-d₆) δ 2.77 (d, *J*=4.8 Hz, 3H), 5.17 (br s, 2H), 6.64, 6.86 (AA'BB' quartet, *J*=8.4 Hz, 4H), 7.06 (dd, *J*=5.5*,* 2.5 Hz, 1H), 7.33 (d, *J*=2.5 Hz, 1H), 8.44 (d, *J*=5.5 Hz, 1H), 8.73 (br d, 1H); HPLC ES-MS *m*/*z* 244 ((M+H)⁺).
Step 3: A solution of 4-chloro-3-(trifluoromethyl)phenyl isocyanate (14.60 g, 65.90 mmol) in CH₂Cl₂ (35 mL) is added dropwise to a suspension of 4-(2-(N-methylcarbamoyl)-4-pyridyloxy)aniline from Step 2; (16.0 g, 65.77 mmol) in CH₂C1₂ (35 mL) at 0 °C. The resulting mixture is stirred at room temp. for 22 h. The resulting yellow solids are removed by filtration, then washed with CH₂Cl₂ (2 x 30 mL) and dried under reduced pressure (approximately 1 mmHg) to afford *N-*(4-chloro-3-(trifluoromethyl)phenyl)-*N*'-(4-(2-(N-methylcarbamoyl)-4-pyridyloxy)phenyl) urea as an off-white solid: mp 207-209 °C; ¹H-NMR (DMSO-d₆) δ 2.77 (d, *J*=4.8 Hz, 3H), 7.16 (m, 3H), 7.37 (d, *J*=2.5 Hz, 1H), 7.62 (m, 4H), 8.11 (d, *J*=2.5 Hz, 1H), 8.49 (d, *J*=5.5 Hz, 1H), 8.77 (br d, 1H), 8.99 (s, 1H), 9.21 (s, 1H); HPLC ES-MS *m*/*z* 465 ((M+H)⁺).

### Example C

N-[2-methoxy-5-(trifluoromethyl)phenyl]-N'-{4-[2-N-methylcarbamoyl-4-pyridyloxy]phenyl} urea 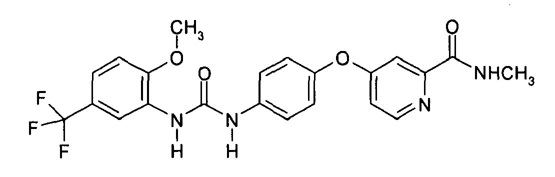
Step 1: 4-Chloro-*N*-methyl-2-pyridinecarboxamide is first synthesized from 4-chloropyridine-2-carbonyl chloride by adding 4-chloropyridine-2-carbonyl chloride HCl salt (7.0 g, 32.95 mmol) in portions to a mixture of a 2.0 M methylamine solution in THF (100 mL) and MeOH (20 mL) at 0 °C. The resulting mixture is stored at 3 °C for 4 h, then concentrated under reduced pressure. The resulting nearly dry solids are suspended in EtOAc (100 mL) and filtered. The filtrate is washed with a saturated NaCl solution (2 x 100 mL), dried (Na₂SO₄) and concentrated under reduced pressure to provide 4-chloro-*N*-methyl-2-pyridinecarboxamide as a yellow, crystalline solid.
Step 2: A solution of 4-aminophenol (9.60 g, 88.0 mmol) in anh. DMF (150 mL) is treated with potassium *tert*-butoxide (10.29 g, 91.7 mmol), and the reddish-brown mixture is stirred at room temp. for 2 h. The contents are treated with 4-chloro-N-methyl-2-pyridinecarboxamide (15.0 g, 87.9 mmol) from Step 1 and K₂CO₃ (6.50 g, 47.0 mmol) and then heated at 80 °C for 8 h. The mixture is cooled to room temp. and separated between EtOAc (500 mL) and a saturated NaCl solution (500 mL). The aqueous phase is back-extracted with EtOAc (300 mL). The combined organic layers are washed with a saturated NaCl solution (4 x 1000 mL), dried (Na₂SO₄) and concentrated under reduced pressure. The resulting solids are dried under reduced pressure at 35 °C for 3 h to afford 4-(2-(*N*-methylcarbamoyl)-4-pyridyloxy)aniline as a light-brown solid. ¹H-NMR (DMSO-d₆) δ 2.77 (d, *J*=4.8 Hz, 3H), 5.17 (br s, 2H), 6.64, 6.86 (AA'BB' quartet, *J*=8.4 Hz, 4H), 7.06 (dd, *J*=5.5*,* 2.5 Hz, 1H), 7.33 (d, *J*=2.5 Hz, 1H), 8.44 (d, *J*=5.5 Hz, 1H), 8.73 (br d, 1H); HPLC ES-MS *m*/*z* 244 ((M+H)⁺).
Step 3: To a solution of 2-methoxy-5-(trifluoromethyl)aniline (0.15 g) in anh CH₂Cl₂ (15 mL) at 0 °C is added CDI (0.13 g). The resulting solution is allowed to warm to room temp. over 1 h, is stirred at room temp. for 16 h, then is treated with 4-(2-(*N-*methylcarbamoyl)-4-pyridyloxy)aniline (0.18 g) from Step 2. The resulting yellow solution is stirred at room temp. for 72 h, then is treated with H₂O (125 mL). The resulting aqueous mixture is extracted with EtOAc (2 x 150 mL). The combined organics are washed with a saturated NaCl solution (100 mL), dried (MgSO₄) and concentrated under reduced pressure. The residue is triturated (90% EtOAc/10% hexane). The resulting white solids are collected by filtration and washed with EtOAc. The filtrate is concentrated under reduced pressure and the residual oil purified by column chromatography (gradient from 33% EtOAc/67% hexane to 50% EtOAc/50% hexane to 100% EtOAc) to give *N*-(2-methoxy-5-(trifluoromethyl)phenyl)-*N*'-(4-(2-(*N*-methylcarbamoyl)-4-pyridyloxy)phenyl) urea as a light tan solid: TLC (100% EtOAc) R*_{f}*0.62; ¹H NMR (DMSO-d₆) δ 2.76 (d, *J*=4.8 Hz, 3H), 3.96 (s, 3H), 7.1-7.6 and 8.4-8.6 (m, 11H), 8.75 (d, *J*=4.8 Hz, 1H), 9.55 (s, 1 H); FAB-MS *m*/*z* 461 ((M+H)⁺).

Listed below are compounds listed in the Tables below which have been synthesized according to the Detailed Experimental Procedures given above:

### Syntheses of Exemplified Compounds

The synthesis of the exemplified compounds is more particularly described in U.S. Patent Application No. 20020042517, published April 11, 2002.

### Tables

The compounds listed in Tables 1-6 below were synthesized according to the general methods shown above, and the more detailed exemplary procedures described in U.S. Patent Application No. 20020042517, published April 11, 2002.

**Table 1. 3-tert-Butylphenyl Ureas**

| | | | | | | | |
|---|---|---|---|---|---|---|---|
| 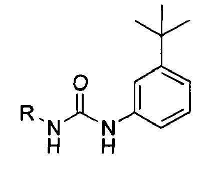 | | | | | | | |

| Entry | R | mp (°C) | HPLC (min.) | TLC R*_{f}* | TLC Solvent System | Mass Spec. [Source] | |
|---|---|---|---|---|---|---|---|
| 1 | 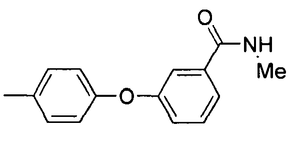 | | | 0.22 | 50% EtOAc / 50% hexane | 418 (M+H)+ (HPLC ES-MS) | |
| 2 | 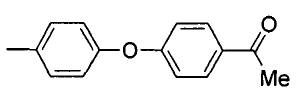 | | | 0.58 | 50% EtOAc / 50% hexane | 403 (M+H)+ (HPLC ES-MS) | |
| 3 | 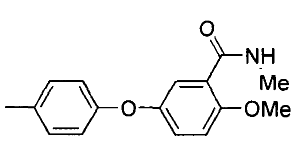 | 133-135 | | 0.68 | 100% EtOAc | 448 (M+H)+ (FAB) | |

**2. 5-tert-Butyl-2-methoxyphenyl Ureas**

| | | | | | | | |
|---|---|---|---|---|---|---|---|
| 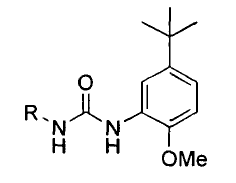 | | | | | | | |

| Entry | R | mp (°C) | HPL C (min.) | TL C R*_{f}* | TLC Solvent System | Mass Spec. [Source] | |
|---|---|---|---|---|---|---|---|
| 4 | 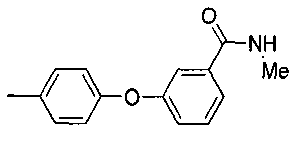 | | 5.93 | | | 448 (M+H)+ (HPLC ES-MS) | |
| 5 | 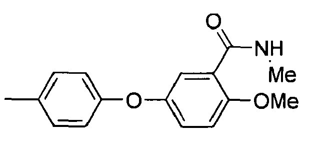 | 120-122 | | 0.67 | 100% EtOAc | 478 (M+H)+ (FAB) | |
| 6 | 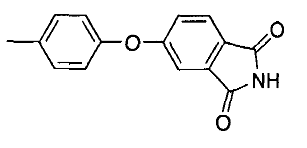 | | | 0.40 | 50% EtOAc / 50% hexane | 460 (M+H)+ (HPLC ES-MS) | |
| 7 | 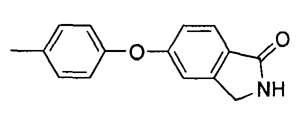 | | | 0.79 | 50% EtOAc / 50% hexane | 446 (M+H)+ (HPLC ES-MS) | |

**Table 3. 5-(Trifluoromethyl)-2-methoxyphenyl Ureas**

| | | | | | | | |
|---|---|---|---|---|---|---|---|
| 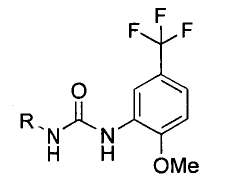 | | | | | | | |

| Entry | R | mp (°C) | HPL C (min.) | TLC R*_{f}* | TLC Solvent System | Mass Spec. [Source] | |
|---|---|---|---|---|---|---|---|
| 8 | 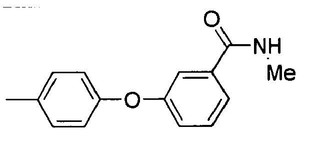 | 250 (dec) | | | | 460 (M+H) + (FAB) | |
| 9 | 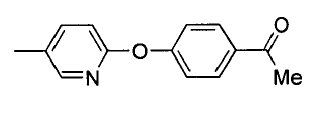 | 206-208 | | 0.54 | 10% MeOH/ 90% CH2Cl2 | 446 (M+H) + (HPLC ES-MS) | |
| 10 | 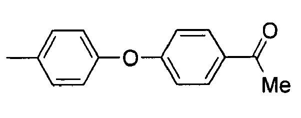 | | | 0.33 | 50% EtOAc/ 50% pet ether | 445 (M+H) + (HPLC ES-MS) | |
| 11 | 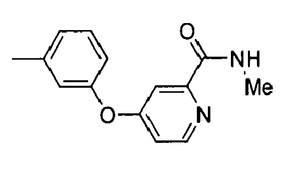 | | | 0.20 | 2% Et3N/ 98% EtOAc | 461 (M+H) + (HPLC ES-MS) | |
| 12 | 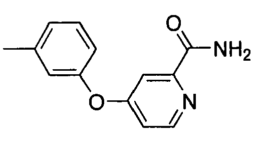 | | | 0.27 | 1% Et3N/ 99% EtOAc | 447 (M+H) + (HPLC ES-MS) | |
| 13 | 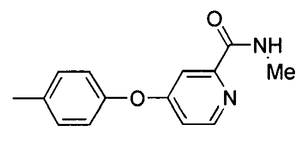 | | | 0.62 | 100% EtOAc | 461 (M+H) + (FAB) | |
| 14 | 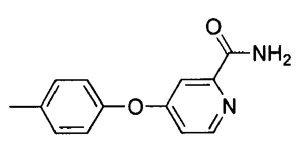 | 114-117 | | 0.40 | 1% Et3N/ 99% EtOAc | 447 (M+H) + (FAB) | |
| 15 | 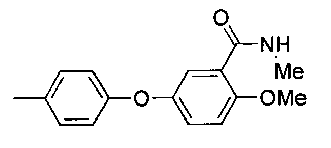 | 232-235 | | 0.54 | 100% EtOAc | 490 (M+H) + (FAB) | |
| 16 | 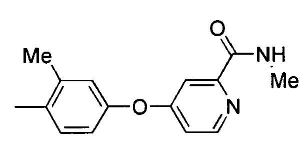 | 210-213 | | 0.29 | 5% MeOH/ 45% EtOAc/ 50% pet ether | 475 (M+H) + (HPLC ES-MS) | |
| 17 | 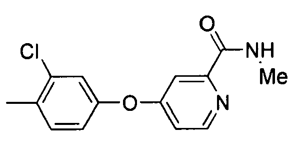 | 187-188 | | 0.17 | 50% EtOAc/ 50% pet ether | 495 (M+H) + (HPLC ES-MS) | |
| 18 | 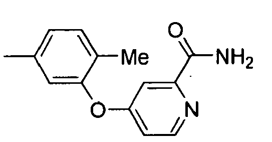 | | | 0.48 | 100% EtOAc | 475 (M+H) + (HPLC ES-MS) | |
| 19 | 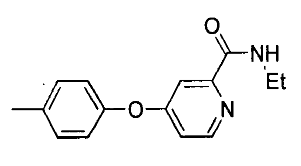 | 194-196 | | 0.31 | 5% MeOH/ 45% EtOAc/ 50% pet ether | 475 (M+H) + (HPLC ES-MS) | |
| 20 | 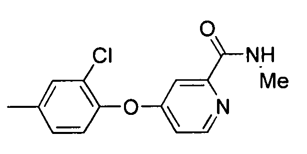 | 214-216 | | 0.25 | 5% MeOH/ 45% EtOAc/ 50% pet ether | 495 (M+H) + (HPLC ES-MS) | |
| 21 | 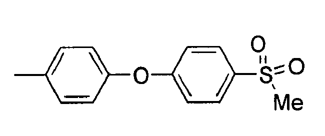 | 208-210 | | 0.30 | 50% EtOAc/ 50% hexane | 481 (M+H) + (HPLC ES-MS) | |
| 22 | 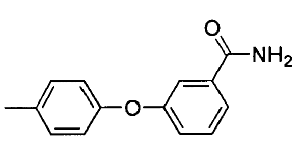 | 188-190 | | 0.30 | 70% EtOAc/ 50% hexane | 447 (M+H) + (HPLC ES-MS) | |
| 23 | 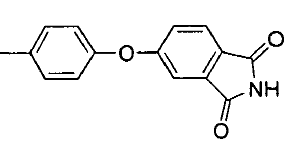 | | | 0.50 | 70% EtOAc/ 30% hexane | 472 (M+H) + (FAB) | |
| 24 | 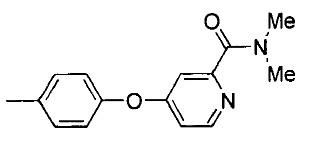 | 203-205 | | 0.13 | 100% EtOAc | 479 (M+H) + (HPLC ES-MS) | |
| 25 | 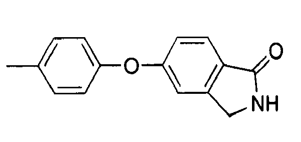 | | | 0.09 | 75% EtOAc/ 25% hexane | 458 (M+H) + (HPLC ES-MS) | |
| 26 | 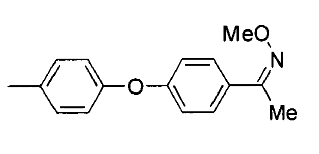 | 169-171 | | 0.67 | 50% EtOAc/ 50% pet ether | 474 (M+H) + (HPLC ES-MS) | |
| 27 | 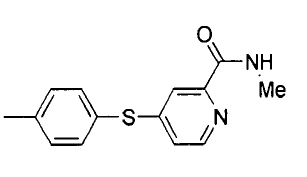 | 218-219 | | 0.40 | 50% EtOAc/ 50% pet ether | 477 (M+H) + (HPLC ES-MS) | |
| 28 | 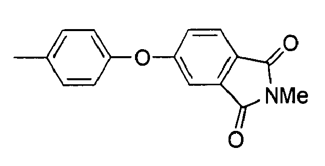 | 212-214 | | 0.30 | 40% EtOAc/ 60% hexane | | |
| 29 | 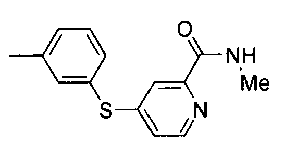 | | | 0.33 | 50% EtOAc/ 50% pet ether | 474 (M+H) + (HPLC ES-MS) | |
| 30 | 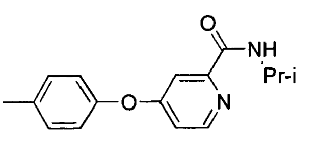 | 210-211 | | | | | |
| 31 | 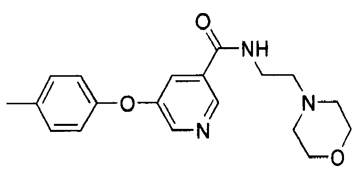 | 210-204 | | 0.43 | 10% MeOH/ CH2Cl2 | | |
| 32 | 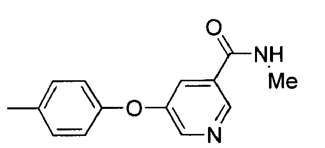 | 247-249 | | 0.57 | 10% MeOH/ CH2Cl2 | | |
| 33 | 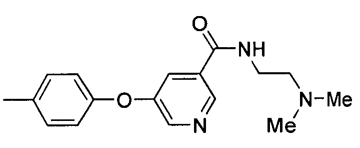 | 217-219 | | 0.07 | 10% MeOH/ CH2Cl2 | | |
| 34 | 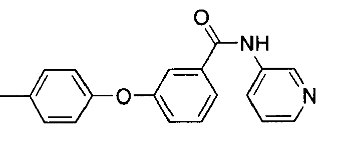 | | | 0.11 | 70% EtOAc/ 30% hexane | | |
| 35 | 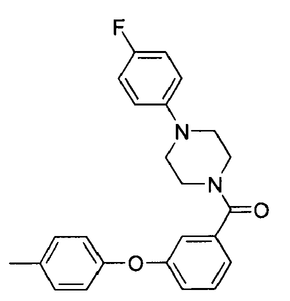 | | | 0.38 | 70% EtOAc/ 30% hexane | | |
| 36 | 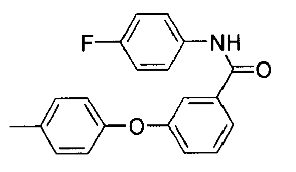 | | | 0.77 | 70% EtOAc/ 30% hexane | | |
| 37 | 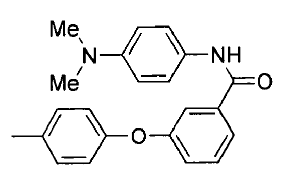 | | | 0.58 | 70% EtOAc/ 30% hexane | | |
| 38 | 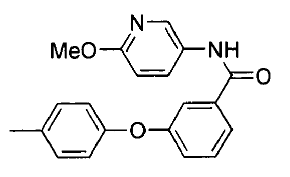 | | | 0.58 | 70% EtOAc/ 30% hexane | | |
| 39 | 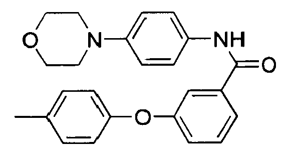 | | | 0.17 | 70% EtOAc/ 30% hexane | | |
| 40 | 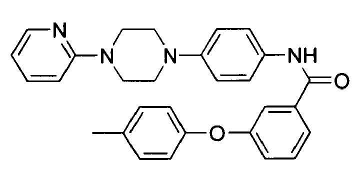 | | | 0.21 | 70% EtOAc/ 30% hexane | | |

**Table 4. 3-(Trifluoromethyl)-4-chlorophenyl Ureas**

| | | | | | | | |
|---|---|---|---|---|---|---|---|
| 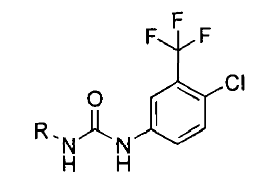 | | | | | | | |

| Entry | R | mp (°C) | HPL C (min. ) | TLC R*_{f}* | TLC Solvent System | Mass Spec. [Source] | |
|---|---|---|---|---|---|---|---|
| 41 | 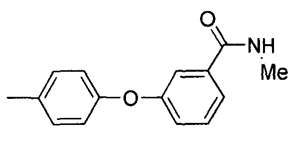 | 163-165 | | 0.08 | 50% EtOAc/ 50% pet ether | 464 (M+H)+ (HPLC ES-MS) | |
| 42 | 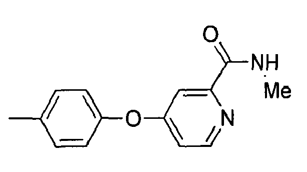 | 215 | | 0.06 | 50% EtOAc/ 50% pet ether | 465 (M+H)+ (HPLC ES-MS) | |
| 43 | 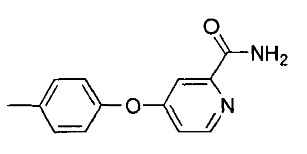 | | | 0.10 | 50% EtOAc/ 50% pet ether | 451 (M+H)+ (HPLC ES-MS) | |
| 44 | 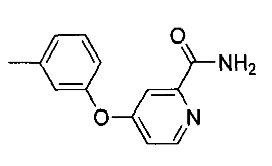 | | | 0.25 | 30% EtOAc/ 70% pet ether | 451 (M+H)+ (HPLC ES-MS) | |
| 45 | 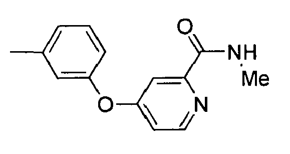 | | | 0.31 | 30% EtOAc/ 70% pet ether | 465 (M+H)+ (HPLC ES-MS) | |
| 46 | 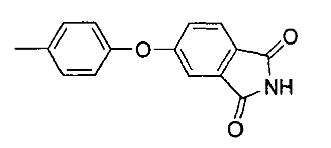 | 176-179 | | 0.23 | 40% EtOAc/ 60% hexane | 476 (M+H)+ (FAB) | |
| 47 | 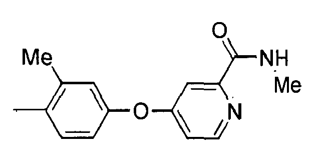 | | | 0.29 | 5% MeOH/ 45% EtOAc/ 50% pet ether | 478 (M+H)+ (HPLC ES-MS) | |
| 48 | 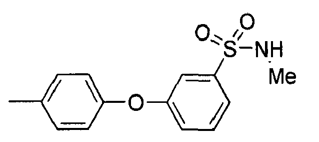 | 206-209 | | | | | |
| 49 | 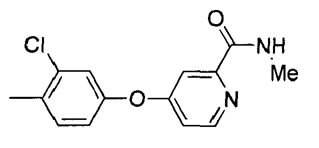 | 147-151 | | 0.22 | 50% EtOAc/ 50% pet ether | 499 (M+H)+ (HPLC ES-MS) | |
| 50 | 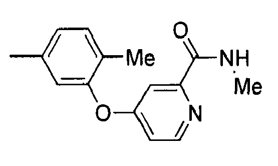 | | | 0.54 | 100% EtOAc | 479 (M+H)+ (HPLC ES-MS) | |
| 51 | 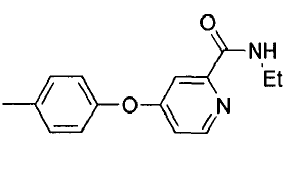 | 187-189 | | 0.33 | 5% MeOH/ 45% EtOAc/ 50% pet ether | 479 (M+H)+ (HPLC ES-MS) | |
| 52 | 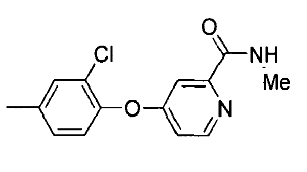 | 219 | | 0.18 | 5% MeOH/ 45% EtOAc/ 50% pet ether | 499 (M+H)+ (HPLC ES-MS) | |
| 53 | 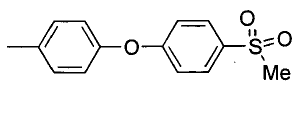 | 246- 248 | | 0.30 | 50% EtOAc/ 50% hexane | 485 (M+H)+ (HPLC ES-MS) | |
| 54 | 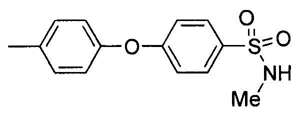 | 196-200 | | 0.30 | 70% EtOAc/ 30% hexane) | 502 (M+H)+ (HPLC ES-MS) | |
| 55 | 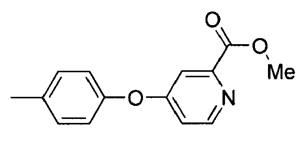 | 228-230 | | 0.30 | 30% EtOAc/ 70% CH2Cl2 | 466 (M+H)+ (HPLC ES-MS) | |
| 56 | 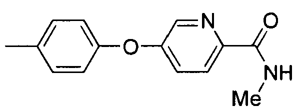 | 238-245 | | | | | |
| 57 | 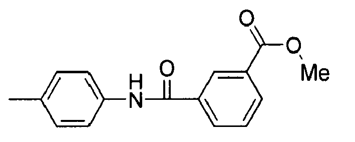 | 221-222 | | 0.75 | 80% EtOAc/ 20% hexane | 492 (M+H)+ (FAB) | |
| 58 | 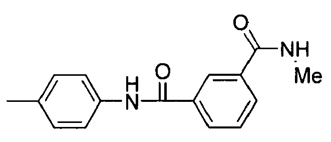 | 247 | | 0.35 | 100% EtOAc | | |
| 59 | 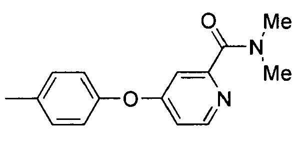 | 198-200 | | 0.09 | 100% EtOAc | 479 (M+H)+ (HPLC ES-MS) | |
| 60 | 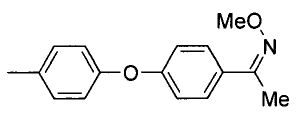 | 158-160 | | 0.64 | 50% EtOAc/ 50% pet ether | | |
| 61 | 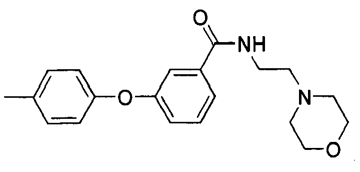 | 195-197 | | 0.39 | 10% MeOH/ CH2Cl2 | | |
| 62 | 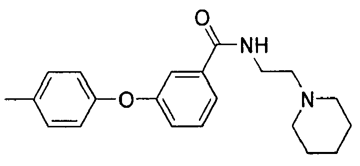 | 170-172 | | 0.52 | 10% MeOH/ CH2Cl2 | | |
| 63 | 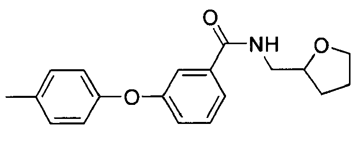 | 168-171 | | 0.39 | 10% MeOH/ CH2Cl2 | | |
| 64 | 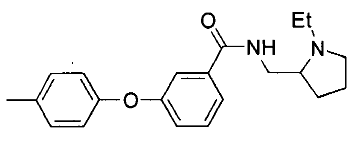 | 176-177 | | 0.35 | 10% MeOH/ CH2Cl2 | | |
| 65 | 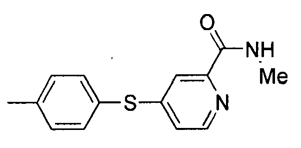 | 130-133 | | | | 487 (M+H)+ (HPLC ES-MS) | |
| 66 | 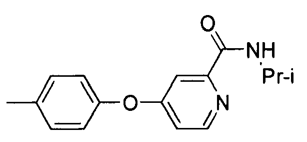 | 155 | | | | | |
| 67 | 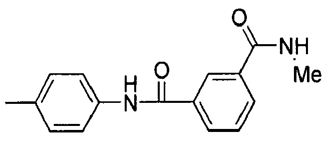 | 225-229 | | 0.23 | 100% EtOAc | | |
| 68 | 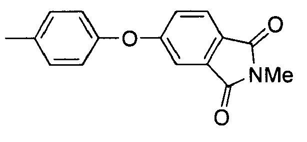 | 234-236 | | 0.29 | 40% EtOAc/ 60% hexane | | |
| 69 | 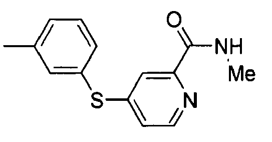 | | | 0.48 | 50% EtOAc/ 50% pet ether | 481 (M+H)+ (HPLC ES-MS) | |
| 70 | 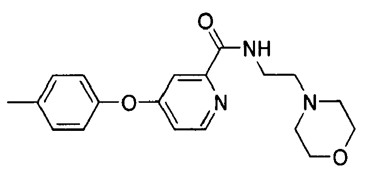 | | | 0.46 | 5% MeOH/ 95% CH2Cl2 | 564 (M+H)+ (HPLC ES-MS) | |
| 71 | 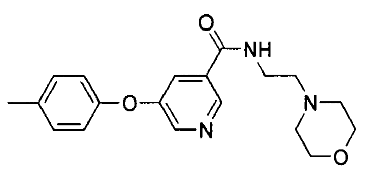 | 199-201 | | 0.50 | 10% MeOH/ CH2C1 2 | | |
| 72 | 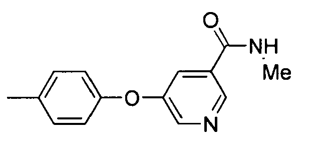 | 235-237 | | 0.55 | 10% MeOH/ CH2C1 2 | | |
| 73 | 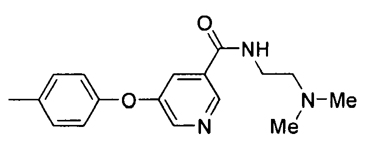 | 200-201 | | 0.21 | 50% MeOH/ CH2C1 2 | | |
| 74 | 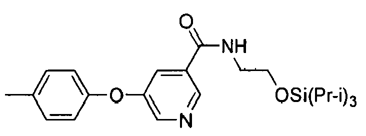 | 145-148 | | | | | |
| 75 | 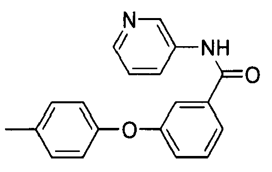 | | | 0.12 | 70% EtOAc/ 30% hexane | 527 (M+H)+ (HPLC ES-MS) | |
| 76 | 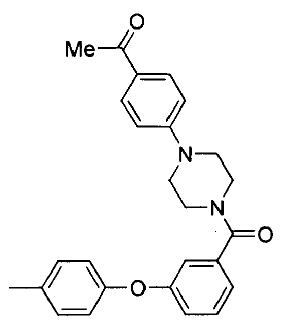 | | | 0.18 | 70% EtOAc/ 30% hexane | | |
| 77 | 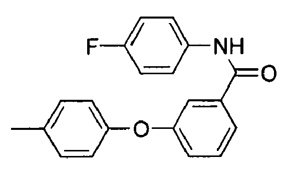 | | | 0.74 | 70% EtOAc/ 30% hexane | | |
| 78 | 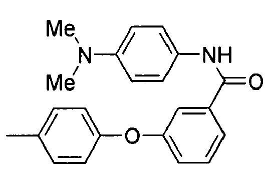 | | | 0.58 | 70% EtOAc/ 30% hexane | | |
| 79 | 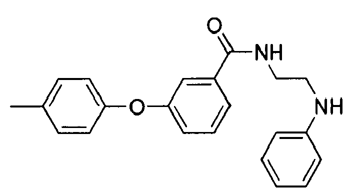 | | | 0.47 | 70% EtOAc/ 30% hexane | 569 (M+H)+ (HPLC ES-MS) | |
| 80 | 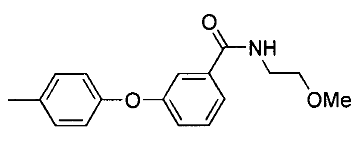 | | | 0.18 | 70% EtOAc/ 30% hexane | 508 (M+H)+ (HPLC ES-MS) | |
| 81 | 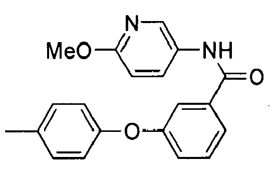 | | | 0.58 | 70% EtOAc/ 30% hexane | 557 (M+H)+ (HPLC ES-MS) | |
| 82 | 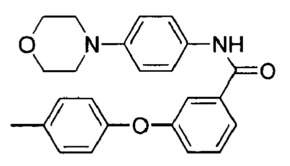 | | | 0.37 | 70% EtOAc/ 30% hexane | 611 (M+H)+ (HPLC ES-MS) | |
| 83 | 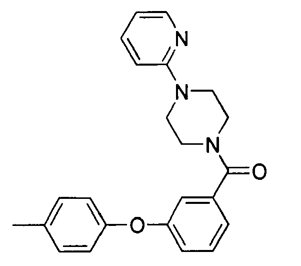 | | | 0.19 | 70% EtOAc/ 30% hexane | | |
| 84 | 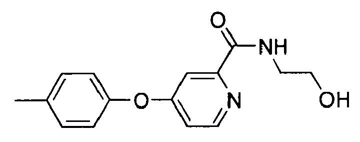 | 179-183 | | | | | |

**Table 5. 3-(Trifluoromethyl)-4-bromophenyl Ureas**

| | | | | | | | |
|---|---|---|---|---|---|---|---|
| 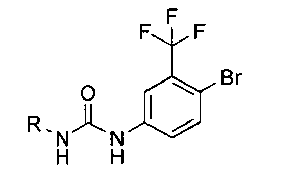 | | | | | | | |

| Entry | R | mp (°C) | HPL C (min.) | TLC R*_{f}* | TLC Solvent System | Mass Spec. [Source] | |
|---|---|---|---|---|---|---|---|
| 85 | 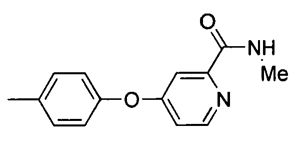 | 186-187 | | 0.13 | 50% EtOAc/ 50% pet ether | 509 (M+H)+ (HPLC ES-MS) | |
| 86 | 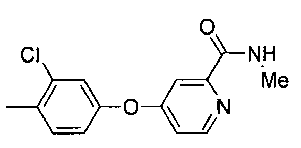 | 150-152 | | 0.31 | 50% EtOAc/ 50% pet ether | 545 (M+H)+ (HPLC ES-MS) | |
| 87 | 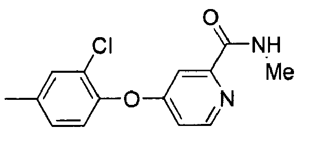 | 217-219 | | 0.16 | 50% EtOAc/ 50% pet ether | 545 (M+H)+ (HPLC ES-MS) | |
| 88 | 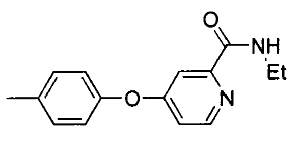 | 183-184 | | 0.31 | 50% EtOAc/ 50% pet ether | 525 (M+H)+ (HPLC ES-MS) | |
| 89 | 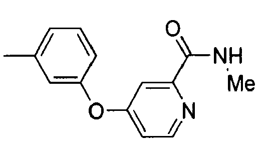 | | | 0.21 | 50% EtOAc/ 50% pet ether | 511 (M+H)+ (HPLC ES-MS) | |
| 90 | 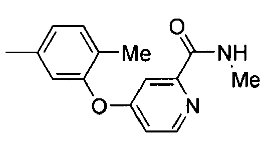 | | | 0.28 | 50% EtOAc/ 50% pet ether | 525 (M+H)+ (HPLC ES-MS) | |
| 91 | 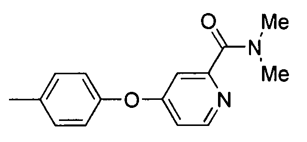 | 214-216 | | 0.28 | 50% EtOAc/ 50% pet ether | 522 (M+H)+ (HPLC ES-MS) | |
| 92 | 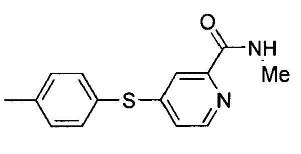 | | | 0.47 | 50% EtOAc/ 50% pet ether | 527 (M+H)+ (HPLC ES-MS) | |
| 93 | 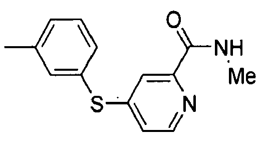 | | | 0.46 | 50% EtOAc/ 50% pet ether | 527 (M+H)+ (HPLC ES-MS) | |
| 94 | 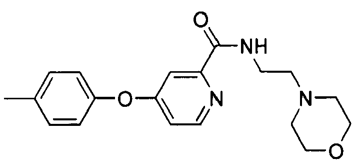 | 145-150 | | 0.41 | 5% MeOH/ 95% CH2Cl2 | | |

**Table 6. 5-(Trifluoromethyl)-4-chloro-2-methoxyphenyl Ureas**

| | | | | | | | |
|---|---|---|---|---|---|---|---|
| 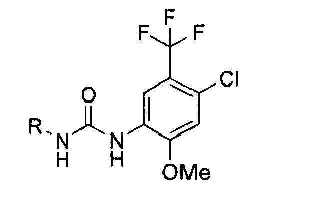 | | | | | | | |

| Entry | R | mp (°C) | HPL C (min. ) | TLC R*_{f}* | TLC Solvent System | Mass Spec. [Source] | |
|---|---|---|---|---|---|---|---|
| 95 | 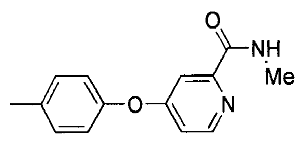 | 140-144 | | 0.29 | 5% MeOH/ 45% EtOAc/ 50% pet ether | 495 (M+H)+ (HPLC ES-MS) | |
| 96 | 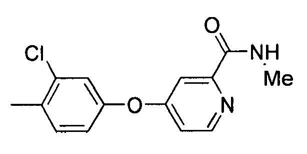 | 244-245 | | 0.39 | 5% MeOH/ 45% EtOAc/ 50% pet ether | 529 (M+H)+ (HPLC ES-MS) | |
| 97 | 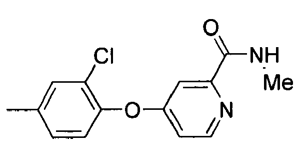 | 220-221 | | 0.25 | 5% MeOH/ 45% EtOAc/ 50% pet ether | 529 (M+H)+ (HPLC ES-MS) | |
| 98 | 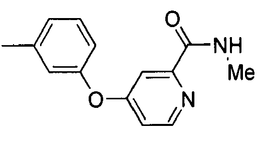 | | | 0.27 | 5% MeOH/ 45% EtOAc/ 50% pet ether | 495 (M+H)+ (HPLC ES-MS) | |
| 99 | 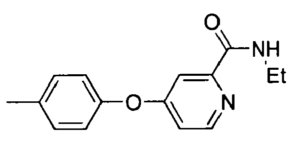 | 180-181 | | 0.52 | 5% MeOH/ 45% EtOAc/ 50% pet ether | 509 (M+H)+ (HPLC ES-MS) | |
| 100 | 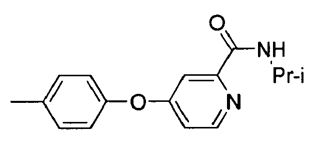 | 162-165 | | | | | |

**Table 7. Additional Ureas**

| Entry | R | mp (°C) | HPL C (min.) | TLC R*_{f}* | TLC Solvent System | Mass Spec. [Source] | |
|---|---|---|---|---|---|---|---|
| 101 | 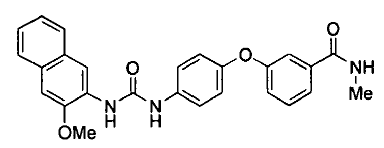 | 162-165 | | | | | |
| 102 | 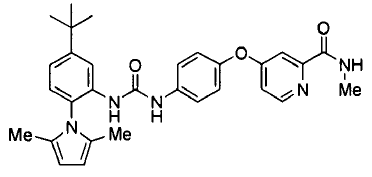 | | | 0.10 | 50% EtOAc/ 50% hexane | 442 (M+H)+ (HPLC ES-MS) | |
| 103 | 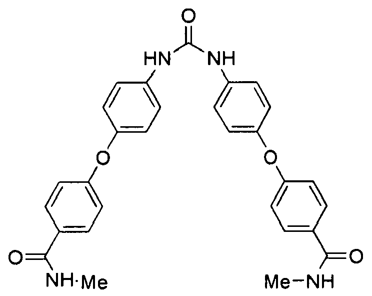 | 125-130 | | 0.24 | 40% EtOAc/ 60% hexane | 512 (M+H)+ (FAB) | |

Selected compounds are named below

From WO 2000/41698

| **Entry No** | **Name** |
|---|---|
| 1 | {3-[4-({[3-(tert-butyl)phenyl]amino}carbonylamino)phenoxy]phenyl}-N-methylcarboxamide |
| 11 | N-[2-methoxy-5-(trifluoromethyl)phenyl]({3-[2-(N-methylcarbamoyl)(4-pyridyloxy)]phenyl}amino)carboxamide |
| 12 | 4-[3-({N-[2-methoxy-5-(trifluoromethyl)phenyl]carbamoyl}amino)phenoxy]pyridine-2-carboxamide |
| 13 | N-[2-methoxy-5-(trifluoromethyl)phenyl]({4-[2-(N-methylcarbamoyl)(4-pyridyloxy)]phenyl}amino)carboxamide |
| 14 | 4-[4-({N-[2-methoxy-5-(trifluoromethyl)phenyl]carbamoyl}amino)phenoxy]pyridine-2-carboxamide |
| 16 | {4-[4-({N-[2-methoxy-5-(trifluoromethyl)phenyl]carbamoyl}amino)-3-methylphenoxy](2-pyridyl)}-N-methylcarboxamide |
| 17 | ({2-chloro-4-[2-(N-methylcarbamoyl)(4-pyridyloxy)]phenyl}amino)-N-[2-methoxy-5-(trifluoromethyl)phenyl]carboxamide |
| 19 | ({4-[2-(N-ethylcarbamoyl)(4-pyridyloxy)]phenyl}amino)-N-[2-methoxy-5-(trifluoromethyl)phenyl]carboxamide |
| 20 | ({3-chloro-4-[2-(N-methylcarbamoyl)(4-pyridyloxy)]phenyl}amino)-N-[2-methoxy-5-(trifluoromethyl)phenyl]carboxamide |
| 22 | 3-[4-({N-[2-methoxy-5-(trifluoromethyl)phenyl]carbamoyl}amino)phenoxy]benzamide |
| 24 | ({4-[2-(N,N-dimethylcarbamoyl)(4-pyridyloxy)]phenyl}amino)-N-[2-methoxy-5-(trifluoromethyl)phenyl]carboxamide |
| 27 | N-[2-methoxy-5-(trifluoromethyl)phenyl]({4-[2-(N-methylcarbamoyl)(4-pyridylthio)]phenyl}amino)carboxamide |
| 29 | N-[2-methoxy-5-(trifluoromethyl)phenyl]({3-[2-(N-methylcarbamoyl)(4-pyridylthio)]phenyl}amino)carboxamide |
| 31 | N-[2-methoxy-5-(trifluoromethyl)phenyl] [(4-{ 5-[N-(2-morpholin-4-ylethyl)carbamoyl](3-pyridyloxy)}phenyl)amino]carboxamide |
| 32 | N-[2-methoxy-5-(trifluoromethyl)phenyl]({4-[5-(N-methylcarbamoyl)(3-pyridyloxy)]phenyl}amino)carboxamide |
| 34 | N-[2-methoxy-5-(trifluoromethyl)phenyl]({4-[3-(N-(3-pyridyl)carbamoyl)phenoxy]phenyl}amino)carboxamide |
| 42 | {4-[4-({[4-chloro-3-(trifluoromethyl)phenyl]amino}carbonylamino)phenoxy](2-pyridyl)}-N-methylcarboxamide |
| 43 | 4-[4-({[4-chloro-3-(trifluoromethyl)phenyl]amino}carbonylamino)phenoxy]pyridi ne-2-carboxamide |
| 44 | 4-[3-({[4-chloro-3-(trifluoromethyl)phenyl]amino}carbonylamino)phenoxy]pyridi ne-2-carboxamide |
| 45 | {[4-chloro-3-(trifluoromethyl)phenyl]amino}-N-{3-[2-(N-methylcarbamoyl)(4-pyridyloxy)]phenyl}carboxamide |
| 47 | {[4-chloro-3-(trifluoromethyl)phenyl]amino}-N-{2-methyl-4-[2-(N-methylcarbamoyl)(4-pyridyloxy)]phenyl}carboxamide |
| 49 | {4-[3-chloro-4-({[4-chloro-3-(trifluoromethyl)phenyl]amino}carbonylamino)phenoxy](2-pyridyl)}-N-methylcarboxamide |
| 51 | N-[4-chloro-3-(trifluoromethyl)phenyl]({4-[2-(N-ethylcarbamoyl)(4-pyridyloxy)]phenyl}amino)carboxamide |
| 61 | {3-[4-({[4-chloro-3-(trifluoromethyl)phenyl]amino}carbonylamino)phenoxy]phenyl }-N-(2-morpholin-4-ylethyl)carboxamide |
| 62 | {3-[4-({[4-chloro-3-(trifluoromethyl)phenyl]amino}carbonylamino)phenoxy]phenyl }-N-(2-piperidylethyl)carboxamide |
| 65 | {4-[4-({[4-chloro-3-(trifluoromethyl)phenyl]amino}carbonylamino)phenylthio](2-pyridyl) }-N-methylcarboxamide |
| 69 | {[4-chloro-3-(trifluoromethyl)phenyl]amino}-N-{3-[2-(N-methylcarbamoyl)(4-pyridylthio)]phenyl}carboxamide |
| 70 | {4-[4-({[4-chloro-3-(trifluoromethyl)phenyl]amino}carbonylamino)phenoxy](2-pyridyl)}-N-(2-morpholin-4-ylethyl)carboxamide |
| 72 | {5-[4-({[4-chloro-3-(trifluoromethyl)phenyl]amino}carbonylamino)phenoxy](3-pyridyl)}-N-methylcarboxamide |
| 75 | N-[4-chloro-3-(trifluoromethyl)phenyl]({4-[3-(N-(3-pyridyl)carbamoyl)phenoxy]phenyl}amino)carboxamide |
| 84 | {4-[4-({[4-chloro-3-(trifluoromethyl)phenyl]amino}carbonylamino)phenoxy](2-pyridyl)}-N-(2-hydroxyethyl)carboxamide |
| 87 | {4-[4-({[4-bromo-3-(trifluoromethyl)phenyl]amino}carbonylamino)-2-chlorophenoxy](2-pyridyl)}-N-methylcarboxamide |
| 88 | N-[4-bromo-3-(trifluoromethyl)phenyl]({4-[2-(N-ethylcarbamoyl)(4-pyridyloxy)]phenyl}amino)carboxamide |
| 89 | {[4-bromo-3-(trifluoromethyl)phenyl]amino}-N-{3-[2-(N-methylcarbamoyl)(4-pyridyloxy)]phenyl}carboxamide |
| 90 | {[4-bromo-3-(trifluoromethyl)phenyl]amino}-N-{4-methyl-3-[2-(N-methylcarbamoyl)(4-pyridyloxy)]phenyl}carboxamide |
| 93 | {[4-bromo-3-(trifluoromethyl)phenyl]amino}-N-{3-[2-(N-methylcarbamoyl)(4-pyridylthio)]phenyl}carboxamide |
| 94 | {4-[4-({[4-bromo-3-(trifluoromethyl)phenyl]amino}carbonylamino)phenoxy](2-pyridyl)}-N-(2-morpholin-4-ylethyl)carboxamide |
| 95 | N-[4-chloro-2-methoxy-5-(trifluoromethyl)phenyl]({4-[2-(N-methylcarbamoyl)(4-pyridyloxy)]phenyl}amino)carboxamide |
| 96 | N-[4-chloro-2-methoxy-5-(trifluoromethyl)phenyl]({2-chloro-4-[2-(N-methylcarbamoyl)(4-pyridyloxy)]phenyl}amino)carboxamide |
| 97 | N-[4-chloro-2-methoxy-5-(trifluoromethyl)phenyl]({3-chloro-4-[2-(N-methylcarbamoyl)(4-pyridyloxy)]phenyl}amino)carboxamide |
| 98 | N-[4-chloro-2-methoxy-5-(trifluoromethyl)phenyl]({3-[2-(N-methylcarbamoyl)(4-pyridyloxy)]phenyl}amino)carboxamide |
| 99 | N-[4-chloro-2-methoxy-5-(trifluoromethyl)phenyl]({4-[2-(N-ethylcarbamoyl)(4-pyridyloxy)]phenyl}amino)carboxamide |
| | |

The compounds listed below are suitable for use in this invention and their synthesis is described with greater particularity in WO 2002/85859

| **Entry No** | **Name** |
|---|---|
| 16 | [(4-fluorophenyl)amino]-N-(3-isoquinolyl)carboxamide |
| 25 | N-(2-methoxy(3-quinolyl))[(4-(4-pyridyloxy)phenyl)amino]carboxamide |
| 27 | N-(2-methoxy(3-quinolyl))[(3-(4-pyridylthio)phenyl)amino]carboxamide |
| 28 | N-[1-(4-methylpiperazinyl)(3-isoquinolyl)] [(4-(4-pyridyloxy)phenyl)amino]carboxamide |

and WO 2002/85857

| **Entry No** | **Name** |
|---|---|
| 25 | N-(2-methoxy(3-quinolyl))[(4-(4-pyridyloxy)phenyl)amino]carboxamide |
| 27 | N-(2-methoxy(3-quinolyl))[(3-(4-pyridylthio)phenyl)amino]carboxamide |
| 28 | N-[1-(4-methylpiperazinyl)(3-isoquinolyl)][(4-(4-pyridyloxy)phenyl)amino]carboxamide |

The preceding description, utilize the present invention to its fullest extent. The following preferred specific embodiments are, therefore, to be construed as merely illustrative, and not limitative of the remainder of the disclosure in any way whatsoever. The entire disclosure of all applications, patents and publications, cited above and in the figures are hereby incorporated by reference in their entirety, including U.S. Provisional Application Nos. 60/556,062, filed March 25, 2004, 60/520,399, filed November 17, 2003, and 60/471,735, filed May 20, 2003.

## Claims

1. Medicament comprising an interferon and a compound of Formula I:
B-NH-C(O)-NH-L-M-L¹-(Q)₁₋₃ (I),
wherein
B is 4-chloro(2-trifluoromethyl)phenyl,
L is phenyl substituted with 1 to 4 halogens,
M is -O-,
L' is a 5 and 6 membered monocyclic heteroaryl group, having 1 to 3 heteroatoms independently selected from the group consisting of O, N and S,
each Q is independently C(O)R⁴, C(O)OR⁴ and C(O)NR⁴R⁵;
wherein each of R⁴ and R⁵ is independently selected from the group consisting of:
(a) hydrogen,
(b) C₁-C₅ linear, branched, or cyclic alkyl,
(c) phenyl,
(d) C₁-C₃ alkyl-phenyl,
(e) up to per-halo substituted C₁-C₅ linear or branched alkyl, and
(f) -(CH₂)_{q}-X, where X is a 5 or 6 membered monocyclic heterocyclic ring, containing 1 to 4 atoms selected from oxygen, nitrogen and sulfur, which is saturated, partially saturated, or aromatic, or a 8 to 10 membered bicyclic heteroaryl having 1 to 4 heteroatoms selected from the group consisting of O, N and S;
wherein the variable q is an integer selected from 0, 1, 2, 3, or 4.

2. Medicament comprising an interferon and compound of formula I of claim 1, wherein said interferon is interferon alpha-2a.

3. Medicament comprising an interferon and compound of formula I of claims 1 and 2, wherein the compounds are administered concurrently.

4. Medicament comprising an interferon and compound of formula I of claims 1 and 2, wherein the compounds are administered intermittently.

5. Medicament comprising an interferon and a compound of formula I
B-NH-C(O)-NH-L-M-L¹-(Q)₁₋₃ (I)
of claims 1-4, wherein the compound of formula I is administered orally within hard shell capsules, soft gelatin capsules, or tablets.

6. 5 Medicament comprising an effective amount of an interferon and compound of formula I
B-NH-C(O)-NH-L-M-L₁-(Q)₁₋₃ (I)
wherein
B is 4-chloro(2-trifluoromethyl)phenyl,
L is phenyl substituted with 1 to 4 halogens,
M is -O-,
L' is a 5 and 6 membered monocyclic heteroaryl group, having 1 to 3 heteroatoms independently selected from the group consisting of O, N and S,
each Q is independently C(O)R⁴, C(O)OR⁴ and C(O)NR⁴R⁵;
wherein each of R⁴ and R⁵ is independently selected from the group consisting of:
(a) hydrogen,
(b) C₁-C₅ linear, branched, or cyclic alkyl,
(c) phenyl,
(d) C₁-C₃ alkyl-phenyl,
(e) up to per-halo substituted C₁-C₅ linear or branched alkyl, and
(f) -(CH₂)_{q}-X, where X is a 5 or 6 membered monocyclic heterocyclic ring, containing 1 to 4 atoms selected from oxygen, nitrogen and sulfur, which is saturated, partially saturated, or aromatic, or a 8 to 10 membered bicyclic heteroaryl having 1 to 4 heteroatoms selected from the group consisting of O, N and S;
wherein the variable q is an integer selected from 0, 1, 2, 3, or 4 for the treatment of a cancer in a mammalian subject, or a cell derived therefrom.

7. Medicament of claim 6, wherein said interferon is interferon alpha-2a.

8. Medicament of any of claims 6 to 7, wherein the compounds are administered concurrently.

9. Medicament of any of claims 6 to 7, wherein the compounds are administered intermittently.
